# EUROPEAN PATENT APPLICATION

(11) **EP 4 745 143 A2**
(43) Date of publication of application: **20.05.2026**
(21) Application number: 26168854.3
(22) Date of filing: 01.11.2022
(51) Int. Cl.: C07D 491/22

(54) **ANTIBODY-DRUG CONJUGATE INTERMEDIATE COMPRISING SN38 AND PREPARATION METHOD THEREFOR**

(30) Priority: 02.11.2021 CN 202111285580
(62) Divisional of application: 22836026.9
(71) Applicant: MabPlex International Co., Ltd., Shandong 264006 (CN)
(72) Inventor: HUANG, Changjiang, Yantai, Shandong 264006 (CN); XIONG, Jiukai, Yantai, Shandong 264006 (CN); YAN, Xinxin, Yantai, Shandong 264006 (CN); YU, Hongxia, Yantai, Shandong 264006 (CN)
(74) Representative: De Clercq & Partners

(57) **Abstract**

The present disclosure provides an antibody-drug conjugate intermediate containing SN38 and a preparation method thereof, comprising introducing a biologically active water-soluble group PEG into its linker and structurally modifying PEG. The preparation method is simple in steps and cost-saving, and does not introduce heavy metal ions in reactions with higher environmental friendliness. Moreover, the antibody-drug conjugate intermediate synthesized by the preparation method has higher stability and efficacy compared with antibody-drug conjugate intermediates with SN38 as toxin in the prior art.

## Description

### FIELD

The present disclosure relates to the field of antibody-drug conjugates, and in particular to an antibody-drug conjugate intermediate and a preparation method thereof.

### BACKGROUND

Antibody-drug conjugate (ADC), as a novel biological targeting drug, realizes a powerful combination of the targeting effect of monoclonal antibodies with the cytotoxicity of small molecular drugs, and has now become one of the fastest developing fields of tumor-targeting therapy. The three components of ADC, an antibody, a cytotoxin and a linker, together constitute a targeting drug delivery system, where the antibody provides the targeting effect, the linker ensures the stability of ADC in the process of blood transport, and the toxin exerts its killing effect on cancer cells after reaching the target. At present, there are more than 60 ADC drugs in clinical trials for antitumor therapy. Among them, most of the toxins are tubulin inhibitors, and a small part are DNA inhibitors. DNA inhibitors have two advantages over tubulin inhibitors: 1) DNA inhibitors (picomolar IC50 values) are more active than tubulin inhibitors (sub-nanomolar IC50 values), and have better effects in treating tumors with few antigens; 2) it may kill cancer cells that are not in the division phase, and has advantages in treating solid tumors.

Camptothecin compounds are clinically used topoisomerase 1 (TOP1) inhibitors, and have good clinical effects on slow-growing solid tumors. However, the special structure of camptothecin leads to poor water-solubility and lipid-solubility, which thus must be modified for water-solubility. ADC drugs with camptothecin as warhead provide a new solution for the above deficiencies.

At present, two ADC drugs with camptothecin derivatives as warhead have been approved for marketing, which are Enhertu (trastuzumab deruxtecan) and Trodelvy (Sacituzumab govitecan). They meet great clinical needs in treating tumors, especially malignant tumors. In Enhertu developed by AstraZeneca/Daiichi Sankyo, GGFG tetrapeptide activated by cathepsin B is used as a linker, and a small section of self-cleaved structure is introduced to release Dxd, a derivative of Exatecan. For HER2-positive metastatic breast cancer, 99 patients using Enhertu showed an objective remission rate of 54.5% and a disease control rate of 93.9%. Sacituzumab govitecan has a linker of Mcc-triazole spacer-PEG7-x-lysine-PABC, which breaks down in lysosomes (pH around 5) in cells to release camptothecin (SN38). The clinical phase II results of Sacituzumab govitecan for triple-negative breast cancer showed an effective rate as high as 30% and tumor shrinkage in 69.5% of patients, in the case that triple-negative breast cancer is almost "incurable" clinically. In addition, for small cell lung cancer in which multiple treatments had failed, Sacituzumab govitecan may shrink tumors in 60% of patients. For non-small cell lung cancer in which chemotherapy, targeting therapy and PD-1 treatment had all failed, Sacituzumab govitecan showed a control rate up to 43%. However, in clinical trials, despite the significant therapeutic effect, there are also serious safety problems caused by ADC toxicity, such as hematologic toxicity, neurotoxicity, pulmonary toxicity, skin toxicity, hepatotoxicity, ocular toxicity, metabolic abnormality, and cardiotoxicity. Therefore, improving the safety of ADC drugs is a problem that needs to be highly concerned and solved in the current drug development.

In addition, there is also a problem of difficult removal of metal ion reaction products in the synthesis of the linker and load of some ADCs. For example, CL2A-SN38 used in Sacituzumab govitecan involves click chemistry reaction in the synthesis process (see Chinese Patent Application Publication No. CN102448494A, paragraph [0273] on page 50 of the specification), resulting in the presence of Cu²⁺ in the reaction products, which is not easily to be removed.

Linker plays a vital role in the structure of ADC, and will affect the pharmacokinetic parameters, therapeutic indexes and efficacy of ADC. Linker can maintain the stability of ADC complex in the blood flow. For the design of linker, first the stability must be considered. Since ADC drug has a long half-life, the stability of linker is required to prevent linker from being broken down in the blood to release toxins in advance. If linker is not stable enough in the blood, it will lead to the decomposition of ADC before it enters the tumor cells, in which case the drug will have a reduced effect on tumor and even kill other cells by mistake. Moreover, in the process of internalization of ADC by tumor cells, linker should be able to release cytotoxic drugs rapidly. As a result, linker plays a significant decisive role in the safety and efficacy of the developed ADC drugs. In addition, different structures of linkers also greatly affect the difficulty factor, production cost, and environmental friendliness of the production process of ADC, and greatly affect the quality of ADC drugs, such as the stability of ADC, further affecting the safety of ADC drugs.

### SUMMARY

The present disclosure provides an antibody-drug conjugate intermediate containing SN38 and a preparation method thereof. The preparation method is simple in steps and cost-saving, and does not introduce heavy metal ions in reactions with higher environmental friendliness. Moreover, the antibody-drug conjugate intermediate synthesized by the preparation method has good stability, thereby significantly improving the safety of ADC drugs.

The present disclosure relates to an antibody-drug conjugate intermediate represented by formula (I): 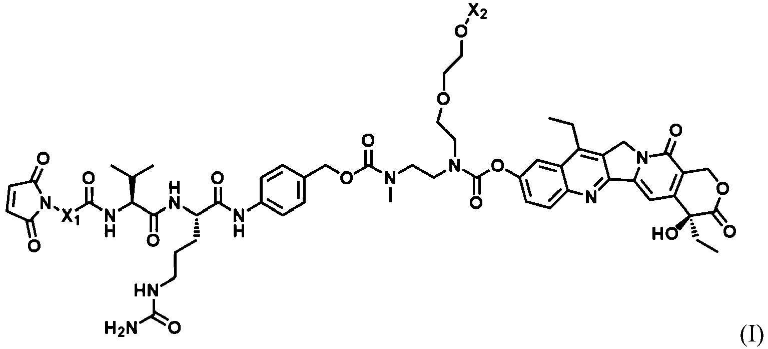 wherein:
X₁ is an alkane chain or a PEG chain,
X₂ is H or -C(O)NR¹R², and
R¹ is selected from the group consisting of hydrogen, halogen, hydroxyl, substituted or unsubstituted C₁₋₆ alkyl, substituted or unsubstituted C₁₋₆ hydroxyalkyl, substituted or unsubstituted C₁₋₆ aminoalkyl, substituted or unsubstituted C₁₋₆ alkoxyl, substituted or unsubstituted C₁₋₆ alkylacyl, and substituted or unsubstituted C₁₋₆ alkylaldehyde;
R² is selected from the group consisting of hydrogen and substituted or unsubstituted C₁₋₆ alkyl; wherein each substituted C₁₋₆ alkyl is optionally substituted with 1-5 substituents independently selected from the group consisting of halogen, hydroxyl, amino, carbonyl, carboxyl, 5-10-membered heterocyclyl and C₁₋₆ haloalkyl, wherein the 5-10-membered heterocyclyl has 1-3 heteroatoms selected from the group consisting of oxygen, nitrogen and sulfur.

Preferably, X₁ is selected from the group consisting of -(CH₂)ₘ- and -(CH₂CH₂O)ₚ-, wherein m is selected from 1, 2, 3, 4, 5, and 6, preferably, m is 5; wherein p is selected from 1, 2, 3, 4, 5, and 6, preferably, P is 2.

Preferably, X₁ is selected from the group consisting of:

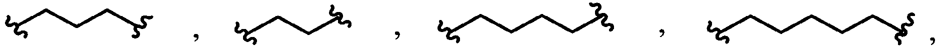

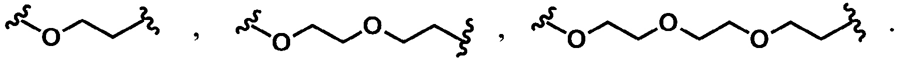

Preferably, R¹ or R² is selected from the group consisting of: H, methyl, ethyl, propyl, butyl, pentyl, heptyl, Cl, Br,

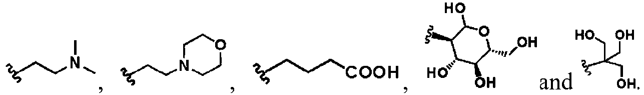

Preferably, X₂ is selected from the group consisting of:

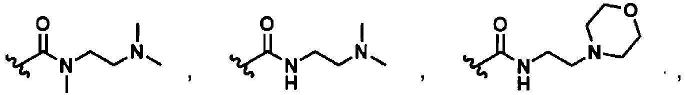

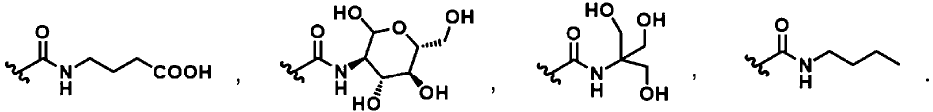

Preferably, the antibody-drug conjugate intermediate has a structure represented by formula (1)-(16): 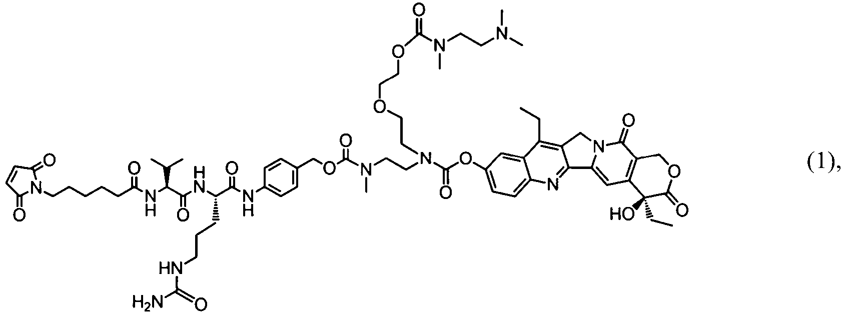 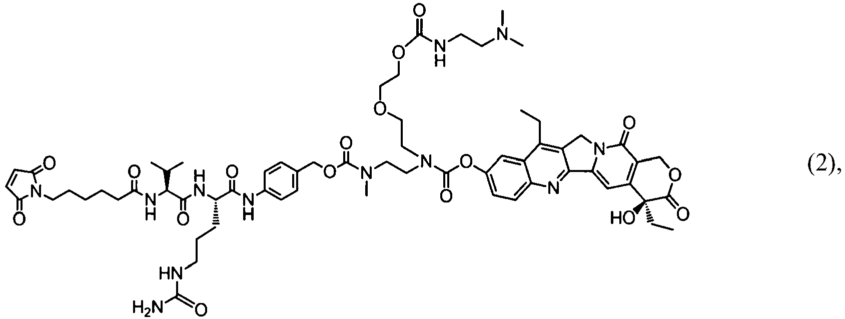 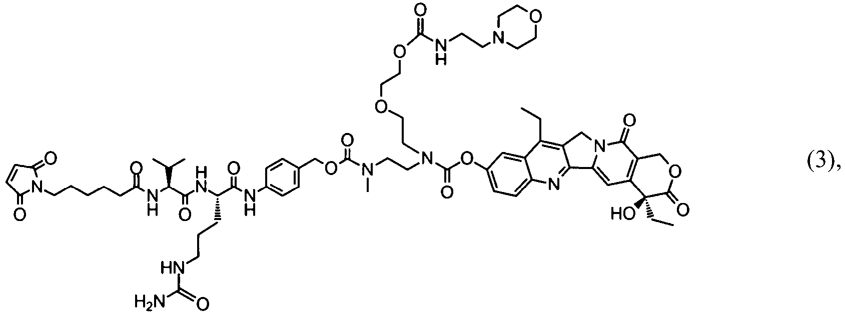 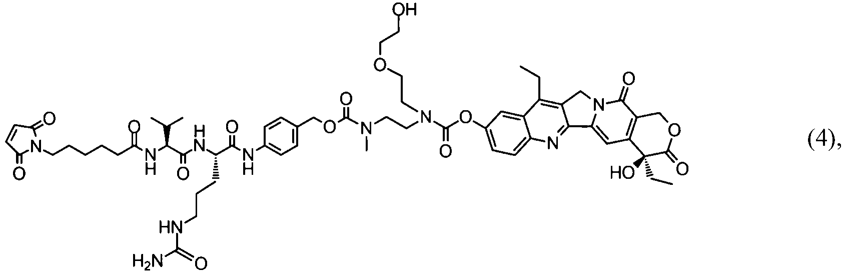 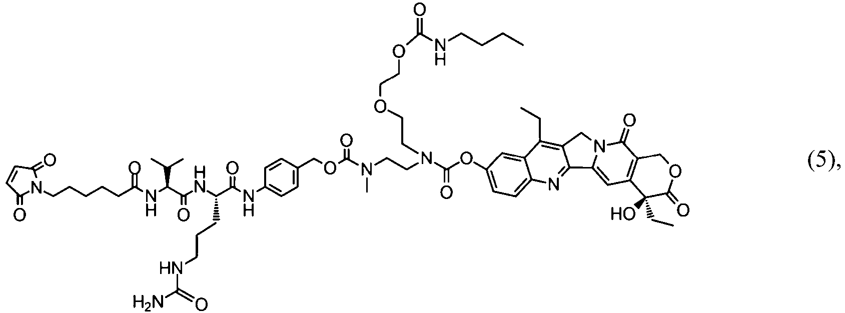 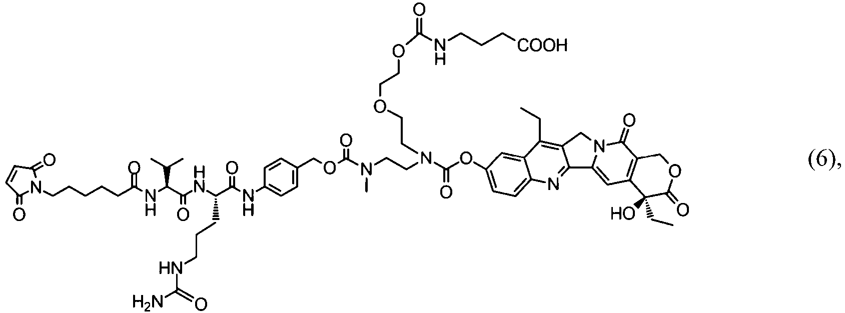 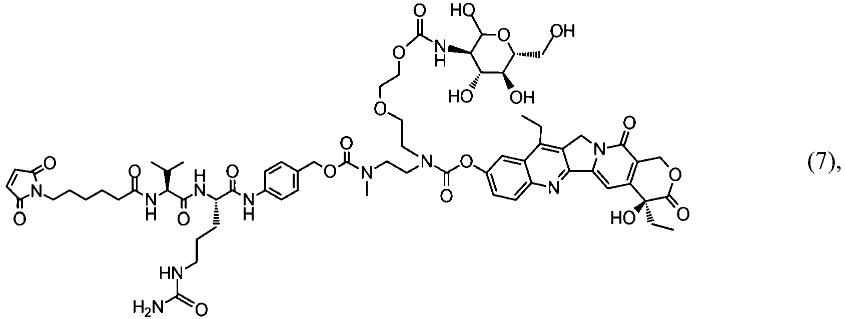 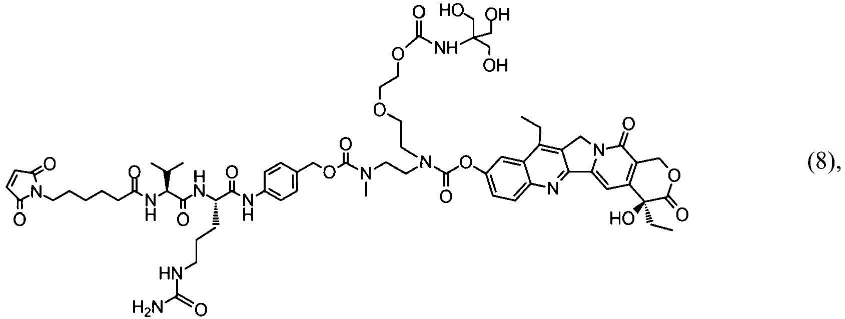 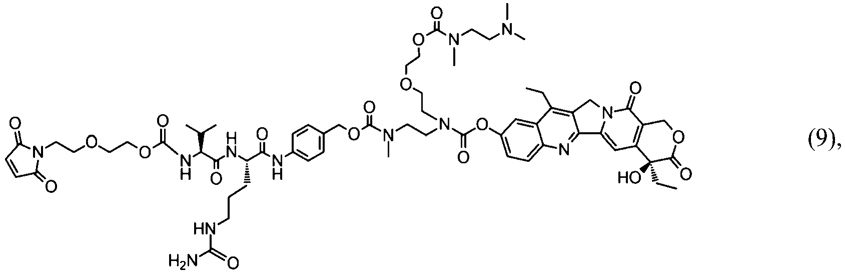 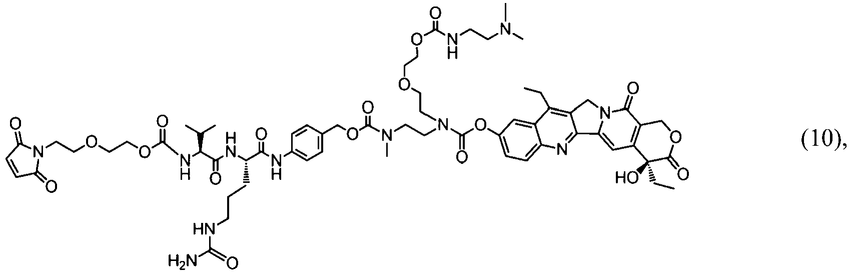 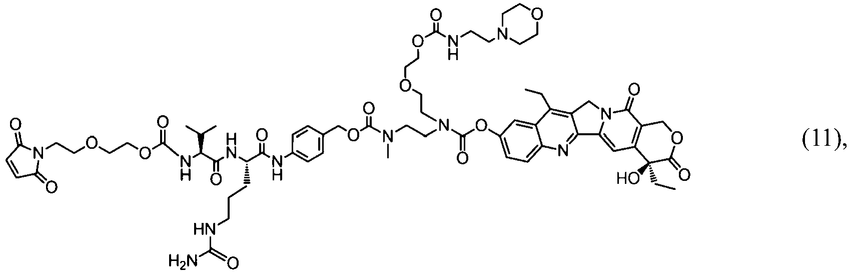 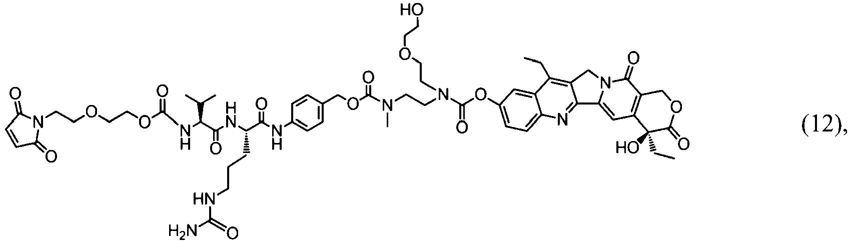 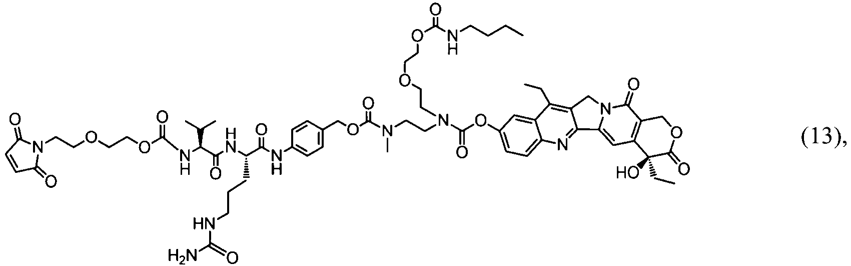 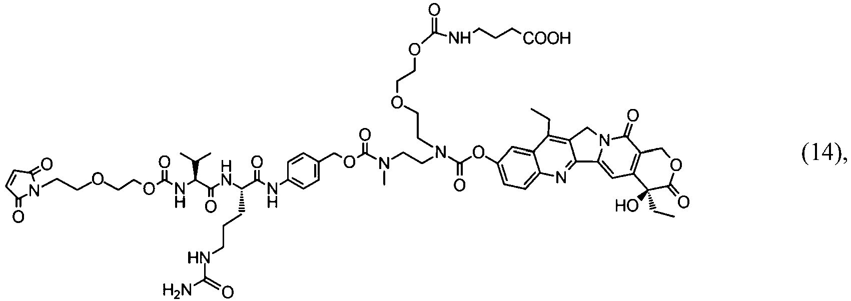 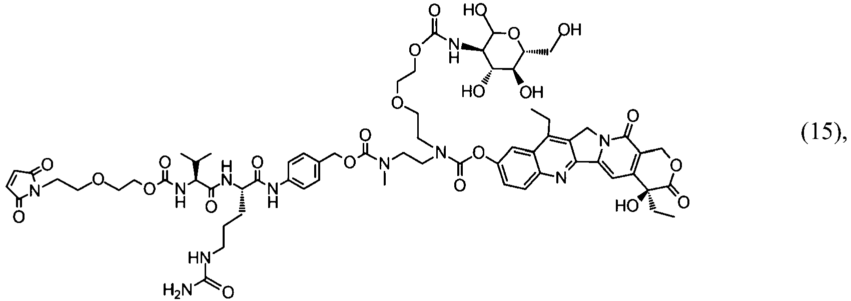 and

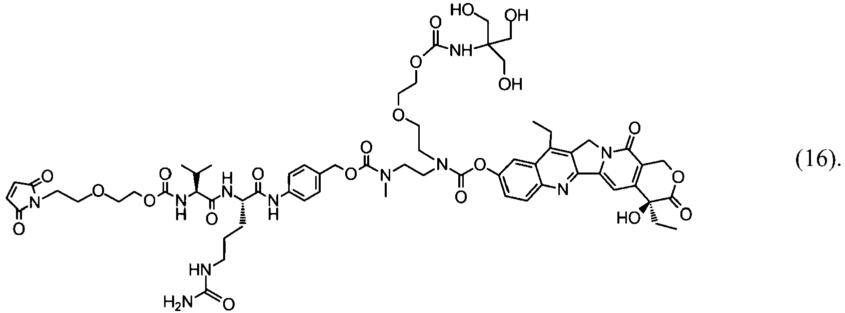

The present disclosure also provides a method for producing an antibody-drug conjugate intermediate.

Further, the antibody-drug conjugate intermediate has a structure of: 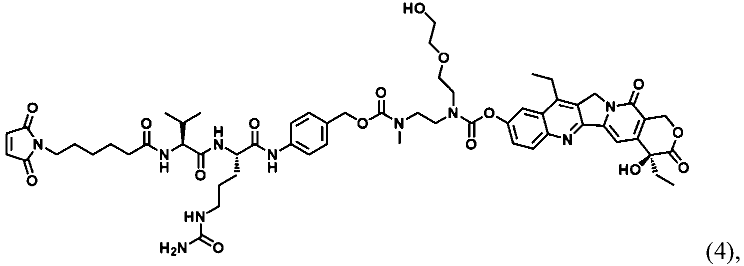 or 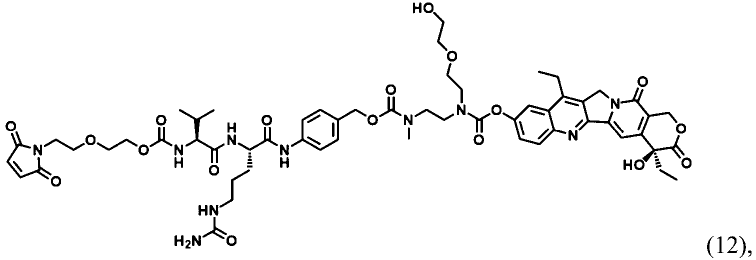 or 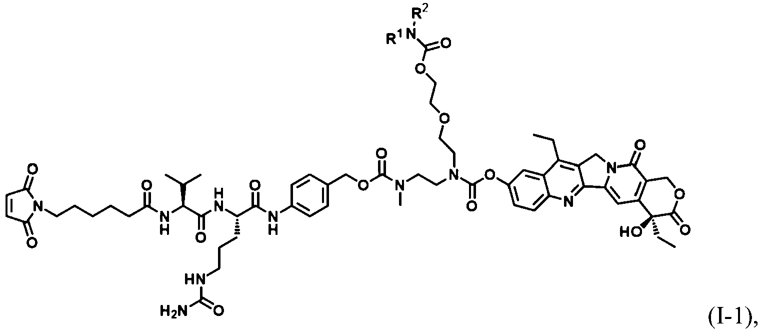 or 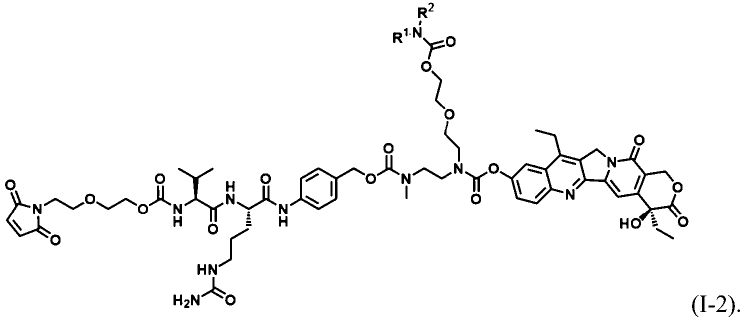
R¹ is selected from the group consisting of hydrogen, substituted or unsubstituted C₁₋₆ alkyl, substituted or unsubstituted C₁₋₆ hydroxyalkyl, substituted or unsubstituted C₁₋₆ aminoalkyl, substituted or unsubstituted C₁₋₆ alkoxyl, substituted or unsubstituted C₁₋₆ alkylacyl, and substituted or unsubstituted C₁₋₆ alkylaldehyde;
R² is selected from the group consisting of hydrogen and substituted or unsubstituted C₁₋₆ alkyl; wherein each substituted C₁₋₆ alkyl is optionally substituted with 1-5 substituents independently selected from the group consisting of halogen, hydroxyl, amino, carbonyl, carboxyl, 5-10-membered heterocyclyl and C₁₋₆ haloalkyl, wherein the 5-10-membered heterocyclyl has 1-3 heteroatoms selected from the group consisting of oxygen, nitrogen and sulfur.

Preferably, R¹ or R² is selected from the group consisting of: H, methyl, ethyl, propyl, butyl, pentyl, heptyl, methoxyl, ethoxyl, Cl, Br,

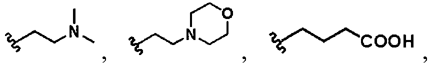

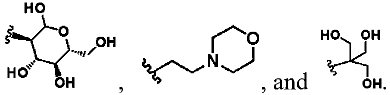

Further, the compound of formula (4) is prepared by:
reaction A: dissolving compound a and compound b in a solvent, stirring at room temperature for an appropriate time, adding a reducing agent at low temperature, stirring for an appropriate time, and then stirring at room temperature overnight to perform a reaction; after the reaction is completed, rotating-drying the solvent, and then performing extraction, drying and purification;
reaction B: dissolving SN38 and DNPC in a solvent, adding an organic base, and stirring at room temperature for an appropriate time to perform a reaction; after the reaction is completed, rotating-drying the solvent, and then performing trituration and filtration;
reaction C: dissolving a product obtained by reaction B in a solvent, adding a product obtained by reaction A and an organic base, stirring at room temperature for an appropriate time to perform a reaction; after the reaction is completed, rotating-drying the solvent, and then performing purification;
reaction D: dissolving a product obtained by reaction C in a solvent, adding an acid, and stirring at low temperature for an appropriate time to perform a reaction; after the reaction is completed, rotating-drying the solvent, dissolving with Mc-VC-PAB-PNP in a solvent, stirring at low temperature for an appropriate time, and adding an organic base to perform a reaction; after the reaction is completed, rotating-drying the solvent, and then performing purification.

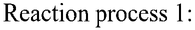

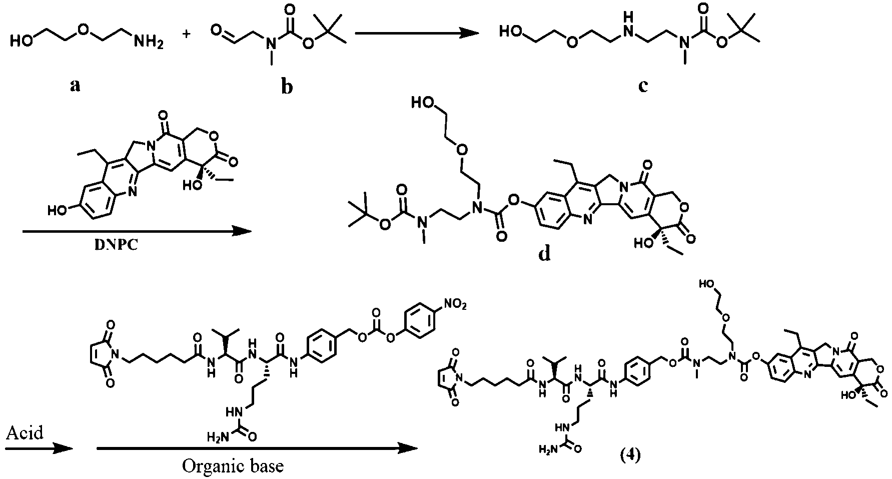

Further, the compound of formula (12) is prepared by:
reaction A: dissolving compound a and compound b in a solvent, stirring at room temperature for an appropriate time, adding a reducing agent at low temperature, stirring for an appropriate time, and then stirring at room temperature overnight to perform a reaction; after the reaction is completed, rotating-drying the solvent, and then performing extraction, drying and purification;
reaction B: dissolving SN38 and DNPC in a solvent, adding an organic base, and stirring at room temperature for an appropriate time to perform a reaction; after the reaction is completed, rotating-drying the solvent, and then performing trituration and filtration;
reaction C: dissolving a product obtained by reaction B in a solvent, adding a product obtained by reaction A and an organic base, stirring at room temperature for an appropriate time to perform a reaction; after the reaction is completed, rotating-drying the solvent, and then performing purification;
reaction D: dissolving a product obtained by reaction C in a solvent, adding an acid, and stirring at low temperature for an appropriate time to perform a reaction; after the reaction is completed, rotating-drying the solvent, dissolving with MP2-VC-PAB-PNP in a solvent, stirring at low temperature for an appropriate time, and adding an organic base to perform a reaction; after the reaction is completed, rotating-drying the solvent, and then performing purification.

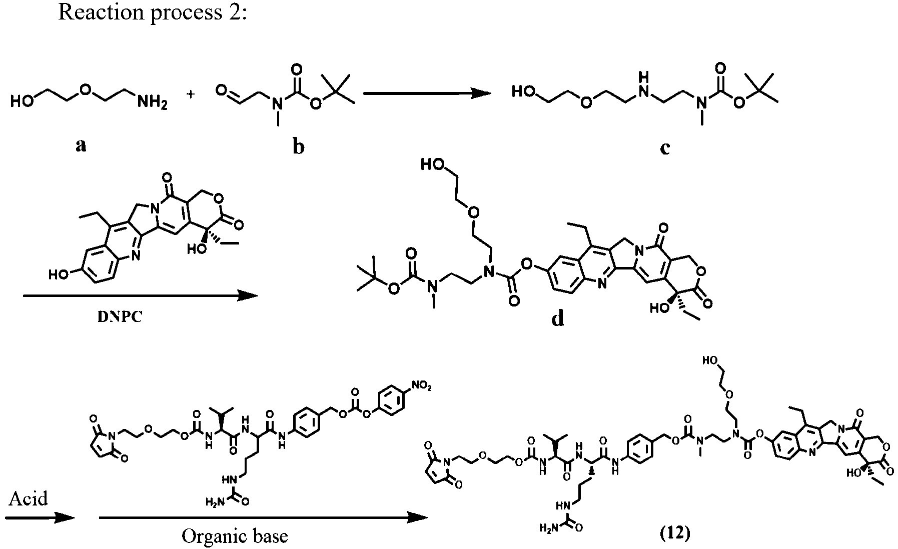

Further, the compound of formula (I-1) is prepared by:
reaction A: dissolving compound a and compound b in a solvent, stirring at room temperature for an appropriate time, adding a reducing agent at low temperature, stirring for an appropriate time, and then stirring at room temperature overnight to perform a reaction; after the reaction is completed, rotating-drying the solvent, and then performing extraction, drying and purification;
reaction B: dissolving SN38 and DNPC in a solvent, adding an organic base, and stirring at room temperature for an appropriate time to perform a reaction; after the reaction is completed, rotating-drying the solvent, and then performing trituration and filtration;
reaction C: dissolving a product obtained by reaction B in a solvent, adding a product obtained by reaction A and an organic base, and stirring at room temperature for an appropriate time to perform a reaction; after the reaction is completed, rotating-drying the solvent, and then performing purification;
reaction D: dissolving a product obtained by reaction C in a solvent, adding bis(p-nitrophenyl) carbonate and an organic base, and stirring at constant temperature for an appropriate time to perform a reaction; after the reaction is completed, rotating-drying the solvent, and then performing purification;
reaction E: dissolving a product obtained by reaction D and an amine in a solvent, adding an organic base, and stirring at low temperature for an appropriate time to perform a reaction; after the reaction is completed, rotating-drying the solvent, and then performing purification;
reaction F: dissolving a product obtained by reaction E in a solvent, adding an acid, and stirring at low temperature for an appropriate time to perform a reaction; after the reaction is completed, rotating-drying the solvent, dissolving with Mc-VC-PAB-PNP in a solvent, stirring at low temperature for an appropriate time, and adding an organic base to perform a reaction; after the reaction is completed, rotating-drying the solvent, and then performing purification.

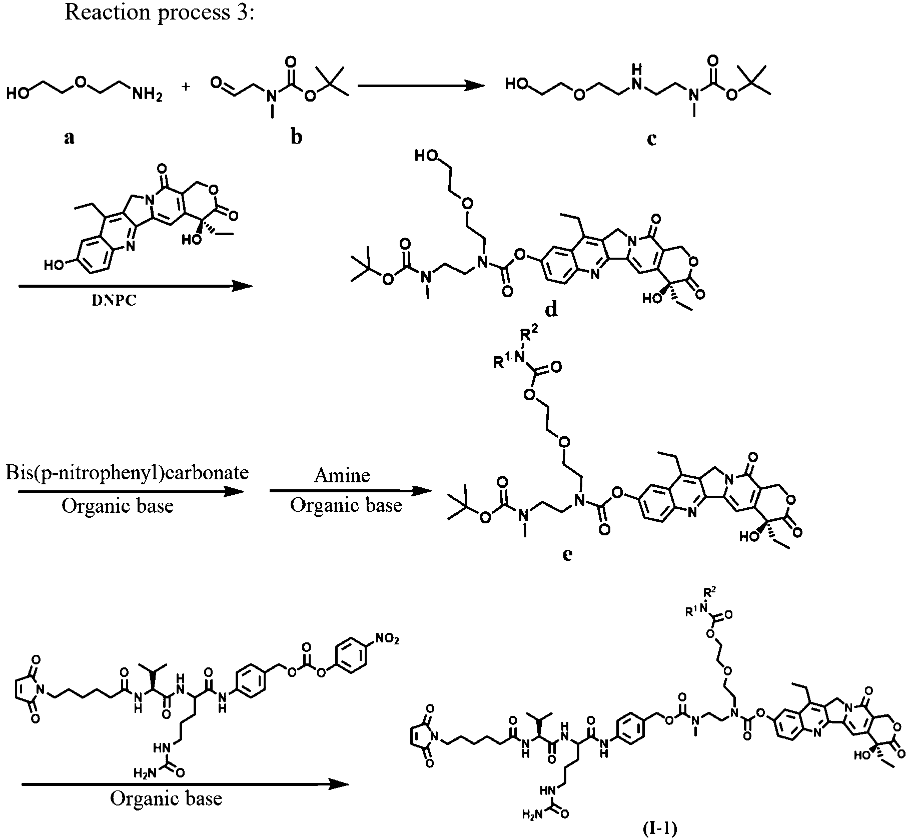

Further, the compound of formula (I-2) is prepared by:
reaction A: dissolving compound a and compound b in a solvent, stirring at room temperature for an appropriate time, adding a reducing agent at low temperature, stirring for an appropriate time, and then stirring at room temperature overnight to perform a reaction; after the reaction is completed, rotating-drying the solvent, and then performing extraction, drying and purification;
reaction B: dissolving SN38 and DNPC in a solvent, adding an organic base, and stirring at room temperature for an appropriate time to perform a reaction; after the reaction is completed, rotating-drying the solvent, and then performing trituration and filtration;
reaction C: dissolving a product obtained by reaction B in a solvent, adding a product obtained by reaction A and an organic base, and stirring at room temperature for an appropriate time to perform a reaction; after the reaction is completed, rotating-drying the solvent, and then performing purification;
reaction D: dissolving a product obtained by reaction C in a solvent, adding bis(p-nitrophenyl) carbonate and an organic base, and stirring at constant temperature for an appropriate time to perform a reaction; after the reaction is completed, rotating-drying the solvent, and then performing purification;
reaction E: dissolving a product obtained by reaction D and an amine in a solvent, adding an organic base, and stirring at low temperature for an appropriate time to perform a reaction; after the reaction is completed, rotating-drying the solvent, and then performing purification;
reaction F: dissolving a product obtained by reaction E in a solvent, adding an acid, and stirring at low temperature for an appropriate time to perform a reaction; after the reaction is completed, rotating-drying the solvent, dissolving with MP2-VC-PAB-PNP in a solvent, stirring at low temperature for an appropriate time, and adding an organic base to perform a reaction; after the reaction is completed, rotating-drying the solvent, and then performing purification.

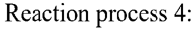

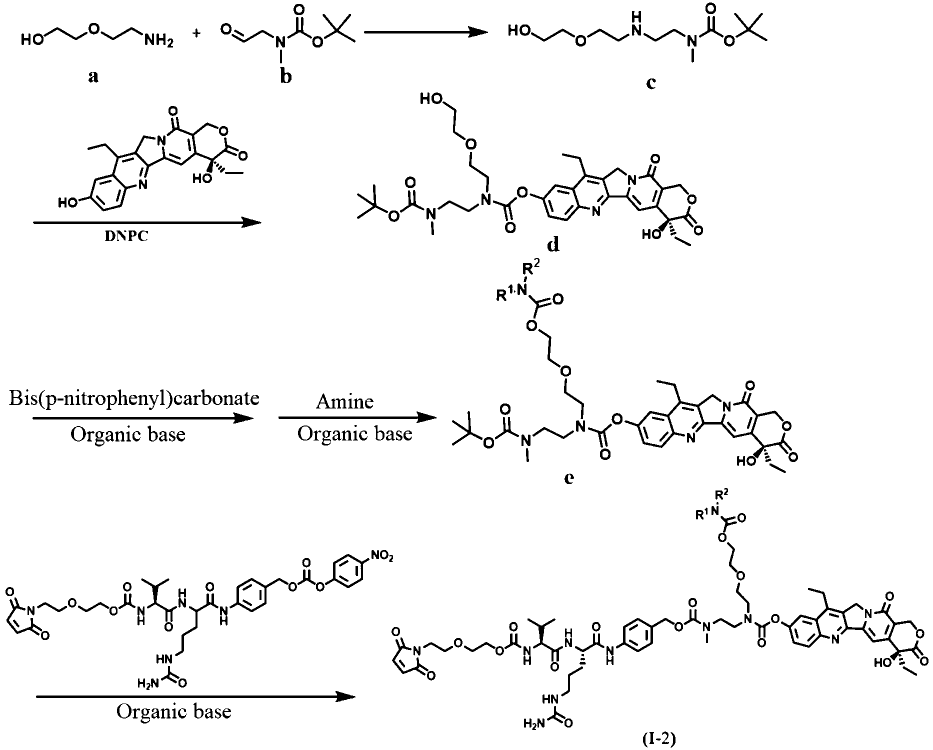

Further, the compound (I-1) is selected from the group consisting of: 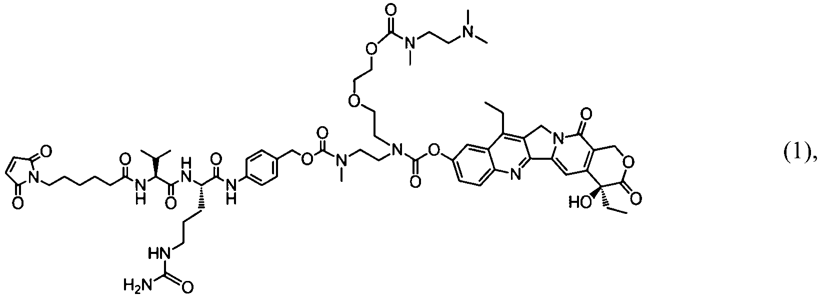 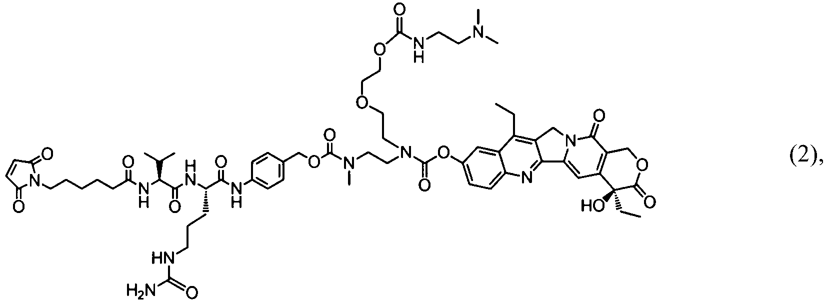 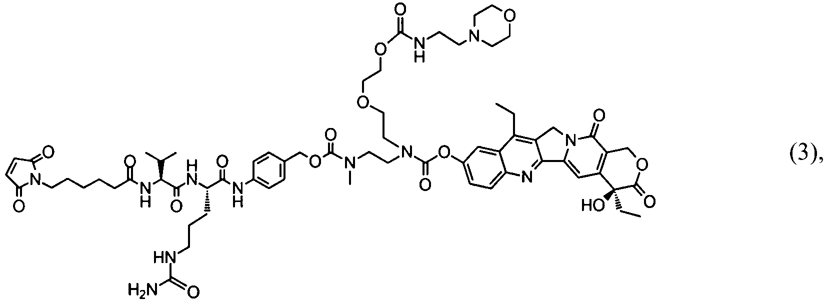 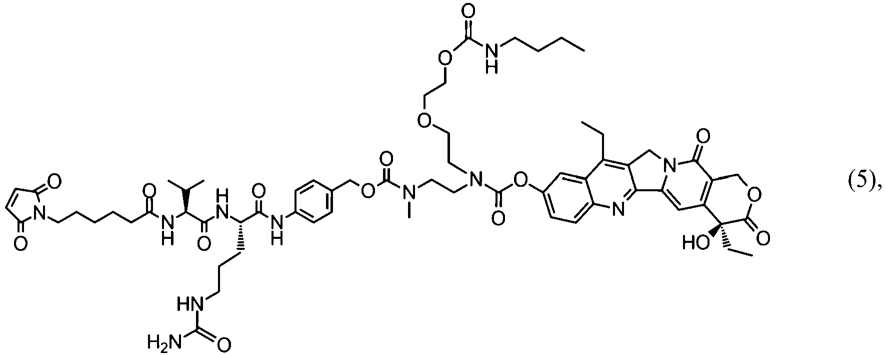 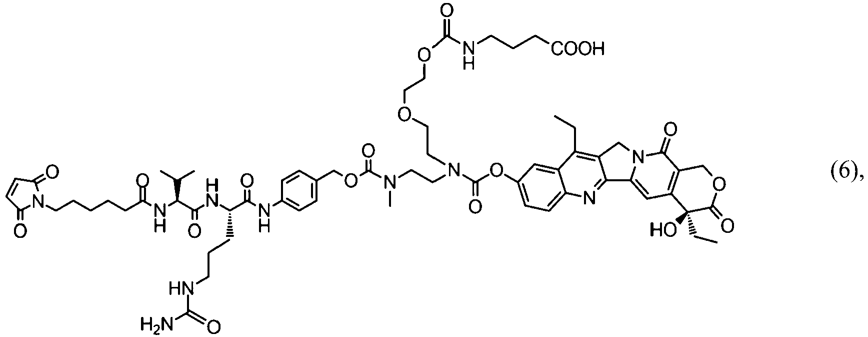 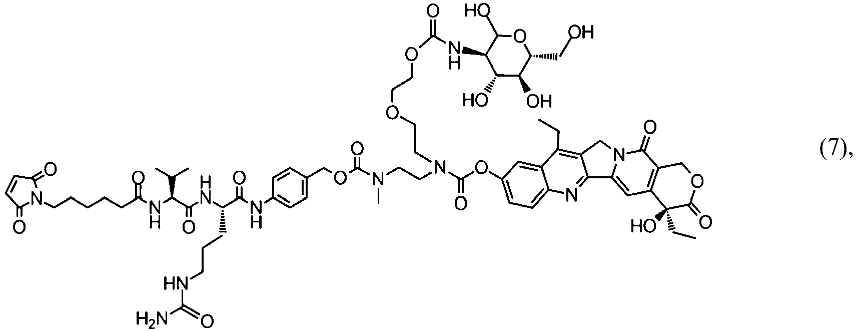 and 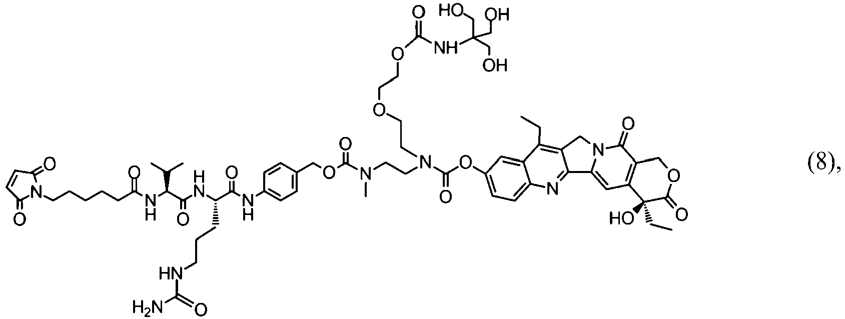 the compound (I-2) is selected from the group consisting of:

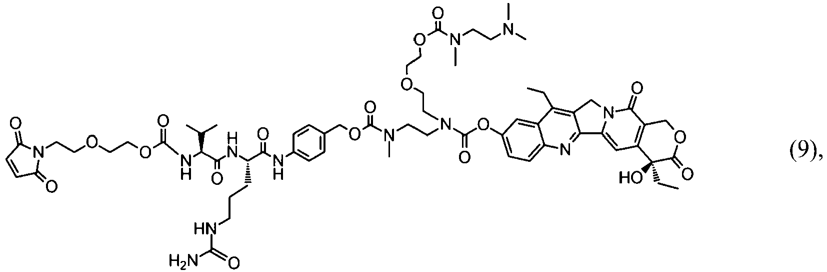

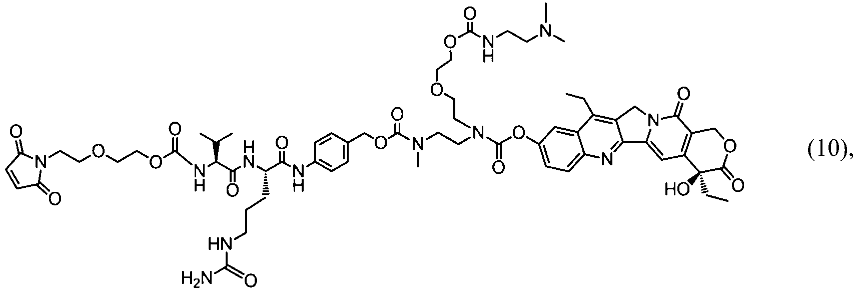

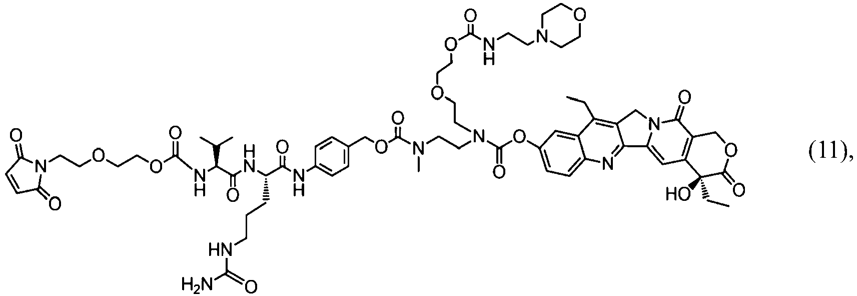

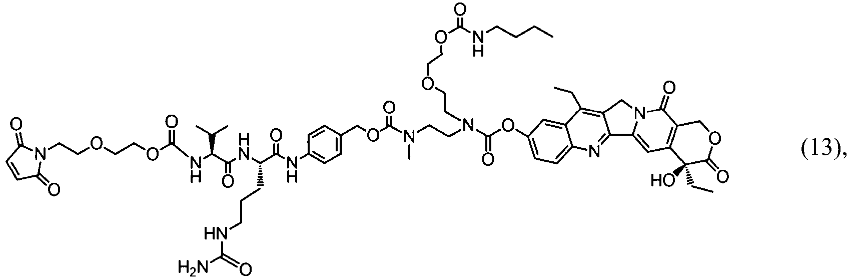

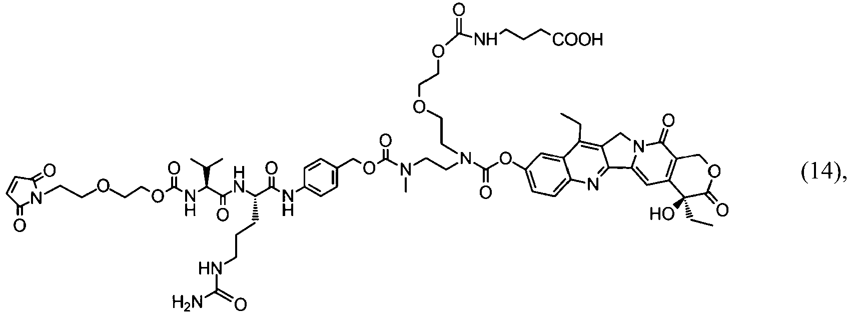

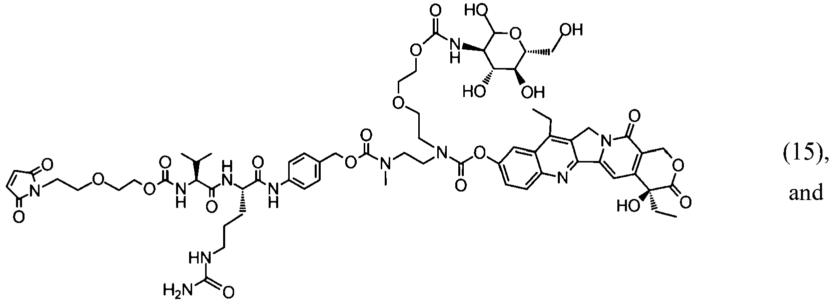

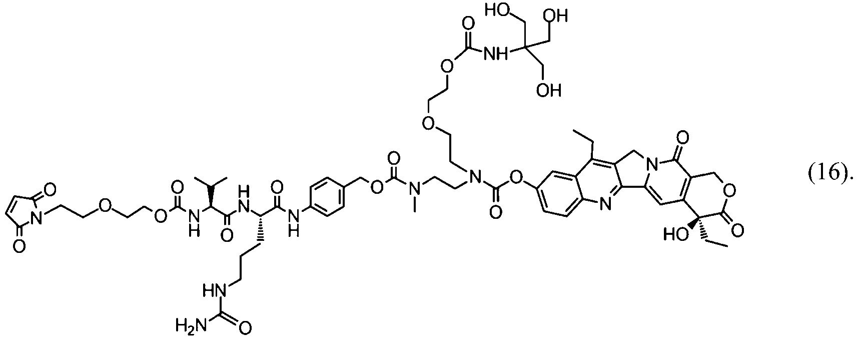

Further, the "at low temperature" in any one of the above-mentioned reaction processes refers to in an ice water bath.

Further, the solvent in any one of the above-mentioned reaction processes may be independently polar solvent and/or non-polar solvent, wherein the polar solvent is selected from the group consisting of THF, DMF, DMA, NMP and a mixture thereof; and the non-polar solvent is selected from the group consisting of dichloromethane, carbon tetrachloride and a mixture thereof.

Further, the organic base in any one of the above-mentioned reaction processes may be independently selected from the group consisting of N,N-diisopropylethylamine, triethylamine, pyridine, and a mixture thereof, preferably one or both of N,N-diisopropylethylamine and pyridine.

Further, the acid is one or two selected from the group consisting of hydrochloric acid, trifluoroacetic acid, and citric acid.

Further, the amine is primary amine or secondary amine.

Further, in the reaction A, the extraction is performed using ethyl acetate, and the purification is performed by column chromatography using dichloromethane and methanol as eluent.

Further, in the reaction B, the trituration is performed using ethyl acetate, hexane, dichloromethane, or a combination thereof.

Further, in the reaction C, the purification is performed by column chromatography using dichloromethane and methanol as eluent.

Further, in the reaction D, the purification is performed by column chromatography using dichloromethane and methanol as eluent.

Further, in the reaction E, the purification is performed by column chromatography using dichloromethane and methanol as eluent.

Further, in the reaction F, the purification is performed by preparative liquid chromatography method using MeCN and 0.1% HCOOH as mobile phase A, and H₂O and 0.1% HCOOH as mobile phase B.

Further, the reactions are all performed under nitrogen protection.

The method for producing an antibody-drug conjugate intermediate (specifically a linker-SN38 covalent conjugate) provided by the present disclosure, in addition to the simple steps, reduces the safety problems caused by heavy metal residues. Moreover, the antibody-drug conjugate prepared by the antibody-drug conjugate intermediate has higher stability *in vivo.*

Furthermore, the inventor surprisingly found that the antibody-drug conjugate prepared by combining the linker and SN38 in the present disclosure can produce significant inhibitory effects on tumor.

### BRIEF DESCRIPTION OF DRAWINGS

FIG. 1 shows the changes in body weight of mice;
FIG. 2 shows the changes in tumor volume;
FIG. 3 shows the relative tumor volume.

### DETAILED DESCRIPTION

### [Abbreviation]

Unless otherwise stated, all abbreviations used in the present disclosure have the same meaning as understood by those of ordinary skill in the art. As used in the present disclosure, commonly used abbreviations and their definitions are shown as follows:

| Abbreviation | Definition |
|---|---|
| DCC | N,N'-Dicyclohexylcarbodiimide |
| EDCI | 1-(3-Dimethylaminopropyl)-3-ethylcarbodiimide hydrochloride |
| DIC | N,N'-Diisopropylcarbodiimide |
| HATU | 2-(7-Azobenzotriazole)-N,N,N',N'-tetramethyluronium hexafluorophosphate |
| HBTU | Benzotriazol-N,N,N',N'-tetramethyluronium hexafluorophosphate |
| HBPIPU | (Benzotriazol-1-yloxy)dipiperidinocarbenium hexafluorophosphate |
| HBPyU | O-(Benzotriazol-1-yl)-N,N,N',N'-dipyrrolyluronium hexafluorophosphate |
| HCTU | 6-Chlorobenzotriazole-1,1,3,3-tetramethyluronium hexafluorophosphate |
| HDMA | 1-[(Dimethylamino)(morpholino)methyl]-3-oxo-1H-[1,2,3]triazolo[4,5-b]pyridine-3-hexafluorophosphate |
| TATU | 2-(7-Azabenzotriazole)-N,N,N',N'-tetramethyluronium tetrafluoroborate |
| TBTU | O-(Benzotriazole)-N,N,N',N'-tetramethyluronium tetrafluoroborate |
| TCTU | O-(6-Chloro-1H-benzotriazol-1-yl)-N,N,N',N'-tetramethyluronium tetrafluoroborate |
| TCFH | N,N,N',N'-Tetramethylchloroformamidinium hexafluorophosphate |
| TDBTU | N,N,N',N'-Tetramethyl-O-(4-carbonyl-3,4-dihydro-1,2,3-benzotriazin-3-yl)uronium tetrafluoroborate |
| TFFH | Fluoro-N,N,N',N'-tetramethyluronium hexafluorophosphate |
| BTFFH | N,N,N',N'-Bis(tetramethylene)fluoroformamidinium hexafluorophosphate |
| TSTU | 2-Succinimidyl-1,1,3,3-tetramethyluronium tetrafluoroborate |
| PyBOP | 1H-Benzotriazole-1-yloxytripyrrolidinophosphonium hexafluorophosphate |
| PyCIOP | Chlorotripyrrolidinophosphonium hexafluorophosphate |
| PyAOP | (3H-1,2,3-triazolo[4,5-b]pyridin-3-oxy)tri-1-pyrrolidinylphosphonium hexafluorophosphate |
| PyCIU | 1-(Chloro-1-pyrrolidinylmethylene)pyrrolidinium hexafluorophosphate |
| DEPBT | 3-(Diethoxy-*o*-acyloxy)-1,2,3-benzotriazin-4-one |
| EEDQ | 2-Ethoxy-1-ethoxycarbonyl-1,2-dihydroquinoline |
| Mc | Maleimidohexanoyl |
| SN38 | Camptothecin |
| DMF | N,N-dimethylformamide |
| DMA | Dimethylacetamide |
| DNPC | Bis(p-nitrophenyl)carbonate |
| DMSO | Dimethyl sulfoxide |
| THF | Tetrahydrofuran |
| DCM | Dichloromethane |
| DIPEA | N,N-diisopropylethylamine |
| TFA | Trifluoroacetic acid |

### [Definition]

Various terms related to various aspects of the specification are used throughout the specification and claims. Unless otherwise indicated, such terms are given their ordinary meaning in the art. Other specifically defined terms should be understood in a manner consistent with the definitions provided herein.

As used herein, the terms "a" and "an" and "the" are used in accordance with standard practice and refer to one or more, unless the context indicates otherwise. Therefore, for example, reference to "an antibody-drug conjugate" includes combinations of two or more antibody-drug conjugates and so on.

It should be understood that wherever the expression of "comprising" is used herein to describe an aspect, similar aspects described by "consisting of ......" and/or "consisting essentially of ......" are also provided.

The term "antibody-drug conjugate" as used herein represents a compound in which an antibody/antibody functional fragment, a linker, and a drug portion are linked together through a chemical reaction, and its structure is usually composed of three portions: an antibody or antibody ligand, a drug portion, and a linker that links the antibody or antibody ligand and the drug. At present, the preparation of antibody-drug conjugates usually comprises two steps: in the first step, the linker and the drug portion are chemically reacted to form a "linker-drug" conjugate, and in the second step, the linker portion of the "linker-drug" conjugate is coupled covalently with the antibody/antibody functional fragment via a sulfhydryl group or an amino group. The term "antibody-drug conjugate intermediate" as used in the present disclosure refers to the "linker-drug" conjugate described above. Further, the term "antibody-drug conjugate intermediate" as used in the present disclosure refers to the covalent conjugate of "linker" and SN38 in general.

The "antibody-drug conjugate" used in the present disclosure is prepared by a common method in the art. For example, the antibody-drug conjugate of the present disclosure is prepared by: preparing a 10 mg/mL solution of antibody with a PBS buffer at pH of 7.4, adding an appropriate equivalent of TCEP, shaking for 1 h to mix well, then adding 5.0 molar equivalents of linker-toxin (*i.e.,* the compound shown in formula 1-formula 16), shaking to mix well for 1 h of reaction, removing the residual small molecules by ultrafiltration after the reaction is completed, and loading into hydrophobic interaction-high performance liquid chromatography (HIC-HPLC) to determine DAR, drug distribution, and antibody ratio.

It is understandable that the present disclosure aims to provide a novel drug conjugate intermediate, and exemplary preparation of multiple ADCs with Heceptin is provided to validate the technical effect. The present disclosure has no limitation on the selection of the antibody, and the structure of specific ADCs is exemplarily provided. ADC-1 has a linker and toxin corresponding to the compound of formula (1), and is prepared with Heceptin by the above mentioned common method for preparing "antibody-drug conjugate" in the art (the following is the same: ADC-2 has a linker and toxin corresponding to the compound of formula (2); ADC-3 has a linker and toxin corresponding to the compound of formula (3); ADC-4 has a linker and toxin corresponding to the compound of formula (4); ADC-5 has a linker and toxin corresponding to the compound of formula (5); ADC-6 has a linker and toxin corresponding to the compound of formula (6); ADC-7 has a linker and toxin corresponding to the compound of formula (7); ADC-8 has a linker and toxin corresponding to the compound of formula (8); ADC-9 has a linker and toxin corresponding to the compound of formula (9); ADC-10 has a linker and toxin corresponding to the compound of formula (10); ADC-11 has a linker and toxin corresponding to the compound of formula (11); ADC-12 has a linker and toxin corresponding to the compound of formula (12); ADC-13 has a linker and toxin corresponding to the compound of formula (13); ADC-14 has a linker and toxin corresponding to the compound of formula (14); ADC-15 has a linker and toxin corresponding to the compound of formula (15); ADC-16 has a linker and toxin corresponding to the compound of formula (16); andADC-17 has a linker and toxin corresponding to CL2A-SN38), wherein q is selected from 1, 2, 3, 4, 5, 6, 7, 8, 9, and 10.

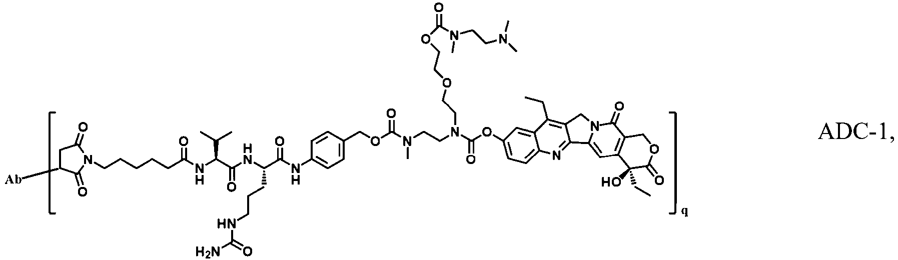

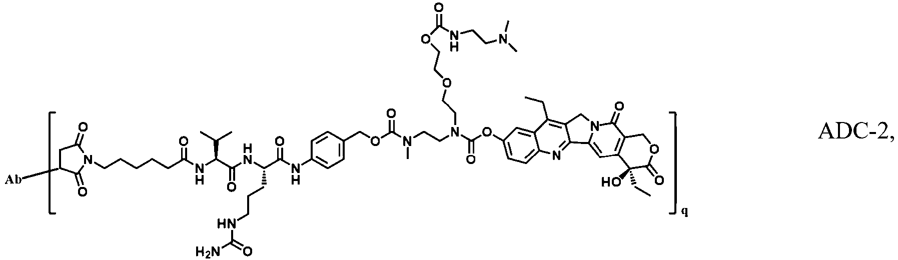

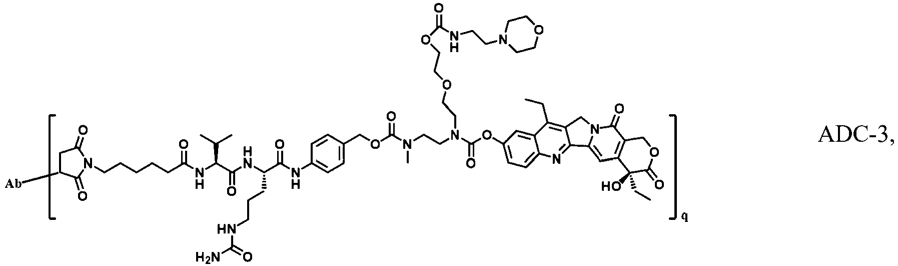

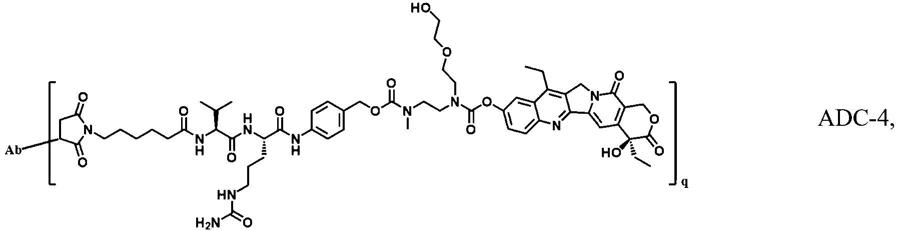

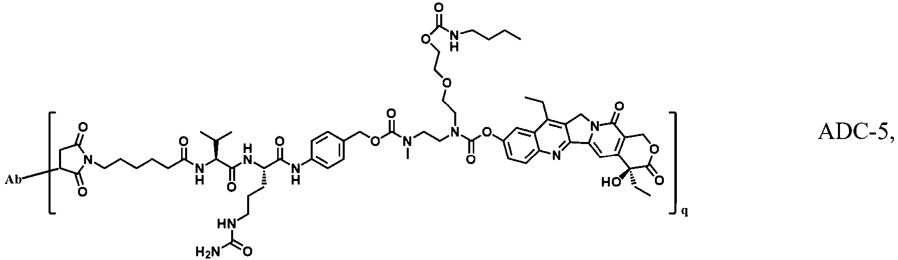

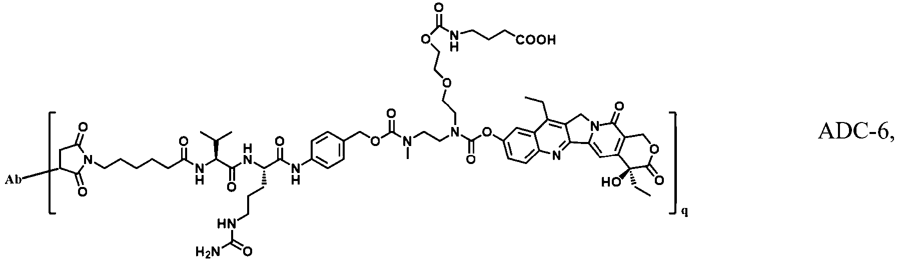

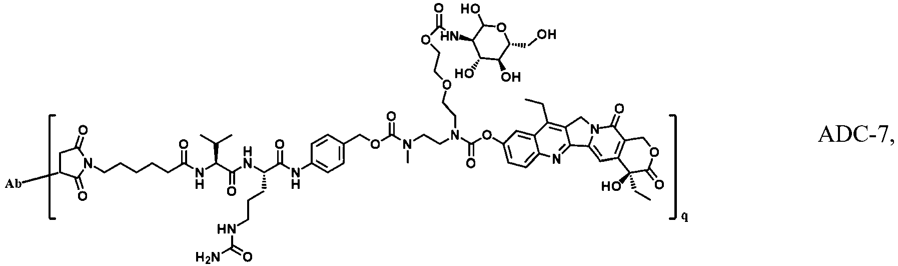

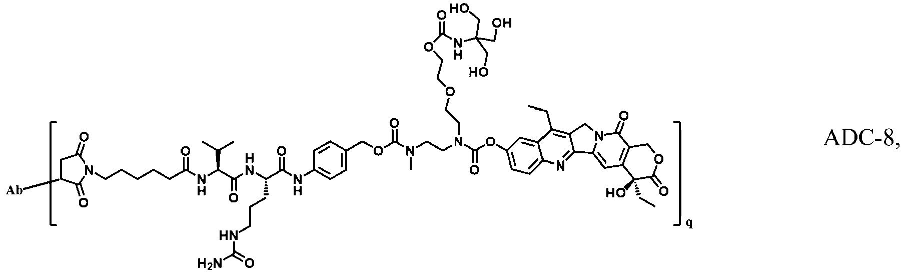

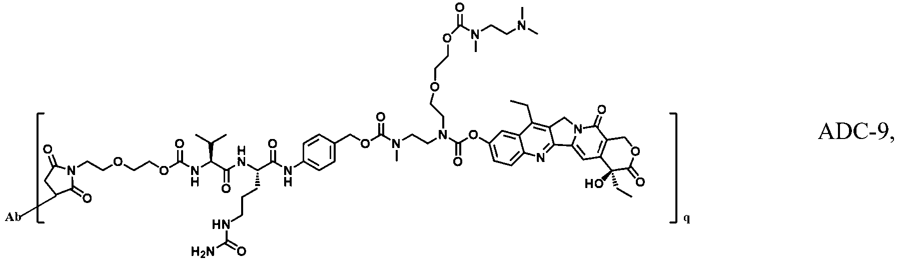

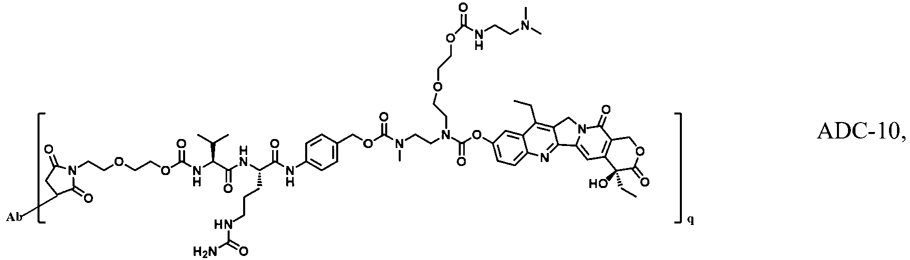

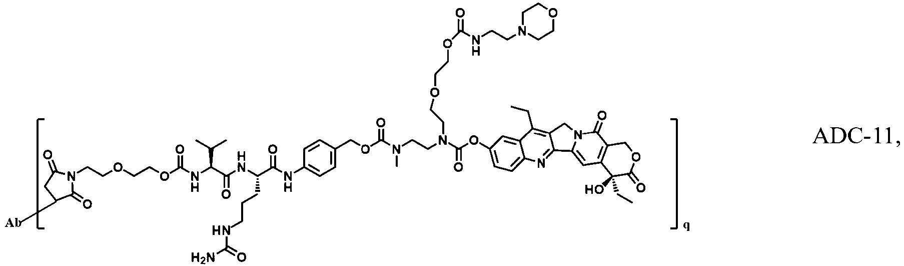

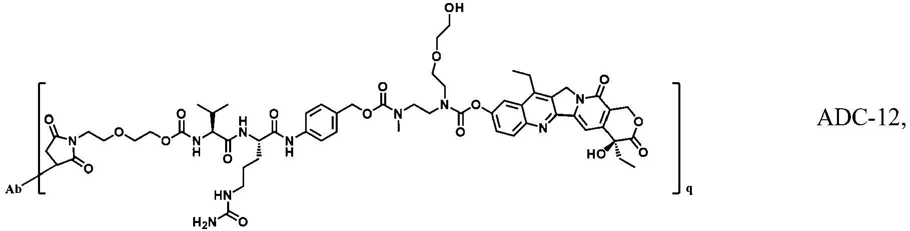

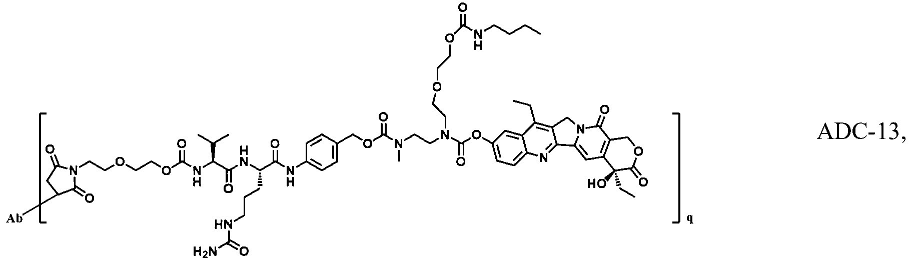

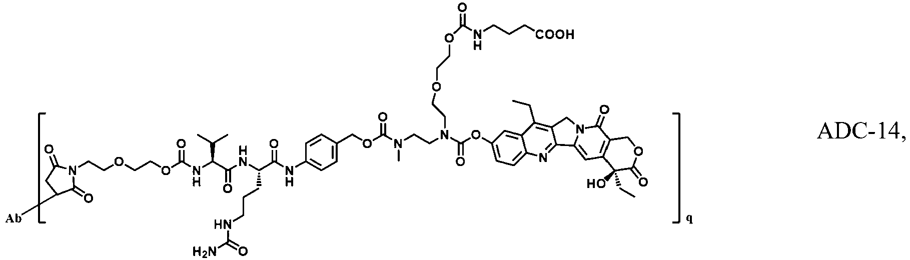

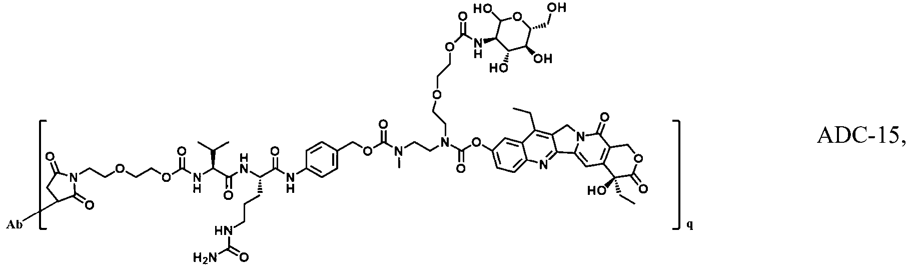

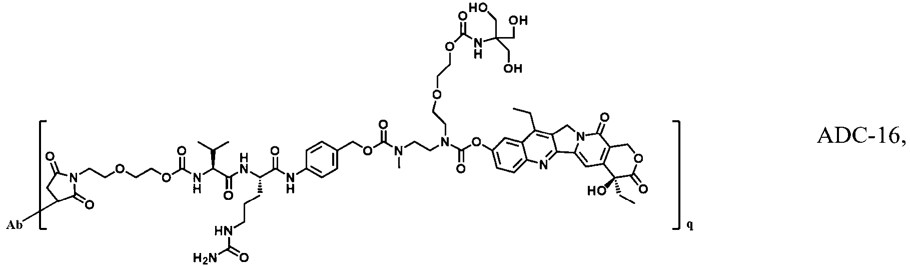

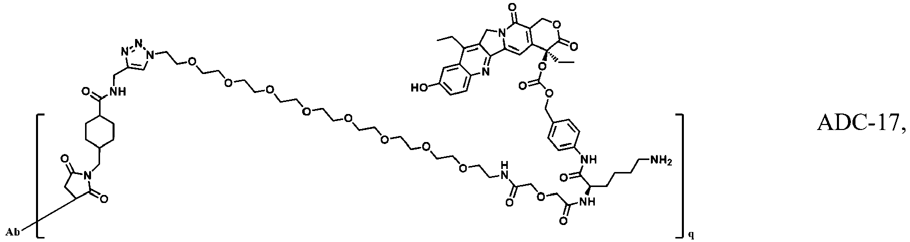

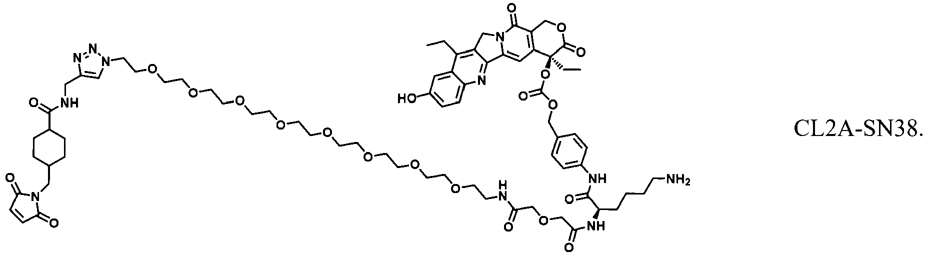

The term "linker" used in the present disclosure refers to a molecule having bifunctional or multifunctional groups, which can react with a protein/antibody molecule and SN38 respectively, and thus act as a "bridge" linking the protein/antibody to SN38. The linker involved in the present disclosure specifically refers to a group containing acyl in the structure.

### [Examples]

The present disclosure is further illustrated below in conjunction with specific examples. It should be understood that these examples are used to illustrate the present disclosure rather than to limit the scope of the present disclosure. The experimental methods in the following examples, which do not specify the specific conditions, are usually carried out according to conventional conditions or according to the conditions recommended by the manufacturer, and reagents of unspecified origin are conventional reagents which are commercially available. All percentages, rates, ratios, or parts are by weight unless otherwise indicated.

Unless otherwise defined, all professional and scientific terms used herein are used in the same sense as that familiar to those skilled in the art. In addition, any methods and materials similar or equivalent to those recited may be applied to the methods of the present disclosure. The preferred examples and materials described herein are for illustrative purposes only.

### Example 1 Preparation of compound of formula (12)

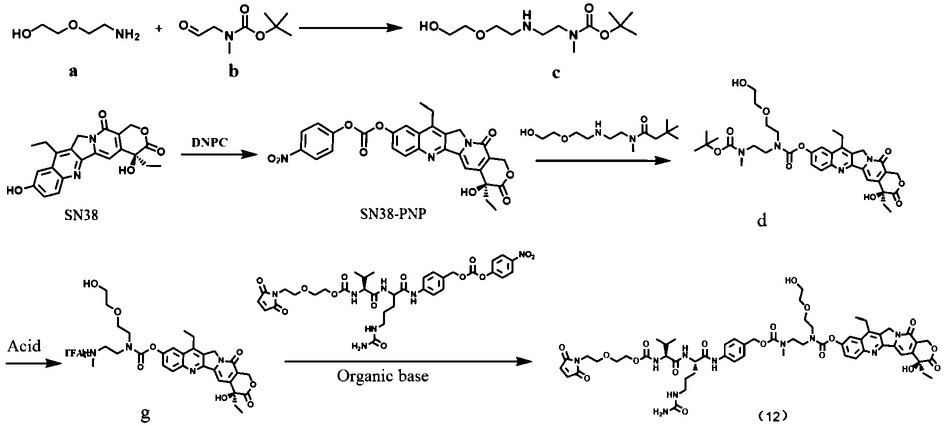

### 1) Synthesis of compound c (2-Boc-N-methylethyl ethylene glycol)

0.6 g of compound a (amino ethylene glycol) and 1.0 g of compound b (N-Boc-acetaldehyde) were dissolved in 20 mL of methanol and stirred at room temperature for 6 h. The reaction system was placed in an ice water bath, added with sodium triacetoxyborohydride, stirred in the ice water bath for 1 h, then returned to room temperature and stirred overnight. The product was detected by LC-MS and showed no UV absorption. The solvent was rotating-dried, and the residue was extracted twice with ethyl acetate, dried over anhydrous Na₂SO₄, rotating-dried, purified by column chromatography using dichloromethane: methanol of 20:1 as eluent, and rinsed to obtain 1.0 g of product with a yield of 66%. LC-MS: (M+H)⁺ 262.6.

### 2) Synthesis of SN38-PNP (10-p-nitrophenyl carbonate-camptothecin)

1.0 g of SN38 (camptothecin) and 1.6 g of DNPC (bis(p-nitrophenyl)carbonate) were dissolved in 100 mL of tetrahydrofuran, added with 2 mL of trimethylamine, and stirred at room temperature for 1.5 h. The reaction was detected to be completed by LC-MS. After the reaction was completed, the solvent was rotating-dried, and the residue was triturated with ethyl acetate: n-hexane of 20 mL: 100 mL, and then filtered to obtain a solid. The solid was triturated with 20 mL of dichloromethane, filtered, and monitored by LC-MS to collect the product to obtain 700 mg of SN38-PNP solid with a yield of 49.3%. LC-MS: (M+H)⁺ 557.4.

### 3) Synthesis of compound d (Boc-N-methylethyl glycol-camptothecin)

250 mg of SN38-PNP was dissolved in 30 mL of N,N-dimethylformamide, added with 235 mg of Boc-DMEDA-PEG and 233 µL of N,N-diisopropylethylamine, and stirred at room temperature for 16 h. The reaction was detected to be completed by LC-MS. After the reaction was completed, the solvent was rotating-dried, and the residue was purified by column chromatography using dichloromethane: methanol of 20:1 as eluent, rinsed, and monitored by LC-MS to collect the product to obtain 213 mg of Boc-DMEDA-PEG-SN38 solid with a yield of 70%. LC-MS: (M+H)⁺ 680.5.

### 4) Synthesis of compound g (10-(2-N-methylethyl ethylene glycol)-camptothecin trifluoroacetate)

73.0 mg of compound d was dissolved in 3 mL of dichloromethane and 3 mL of trifluoroacetic acid, placed in an ice water bath, and stirred at constant temperature for 1 h. The reaction was detected to be completed by LC-MS. After the reaction was completed, the solvent was rotating-dried. The residue was added with 2 mL of toluene and 2 mL of dichloromethane, rotating-dried twice, and monitored by LC-MS to collect the product to obtain SN38-DMEDA·TFA, which was directly used in the next reaction. LC-MS: (M+H)⁺ 580.6.

### 5) Synthesis of compound of formula (12) (maleimide diethoxy-L-valyl-L-citrulline-p-aminobenzyl-(2-N-methylethyl ethylene glycol)-camptothecin)

50 mg of MP2-VC-PAB-PNP was dissolved in 4 mL of N,N-dimethylformamide, placed in an ice water bath to keep the temperature constant, and protected with nitrogen. The reaction system was added with 38 mg of compound g followed by 42 µL of trimethylamine, stirred at constant temperature for 0.5 h, and then returned to room temperature for 0.5 h of reaction. The reaction was detected to be completed by LC-MS. After the reaction was completed, the solvent was rotating-dried at low temperature, and the residue was purified by preparative liquid chromatography using the preparative column of Sun Fire^{®} Prep C18 OBD TM 5µm, 19*250 mm Column, mobile phase A of MeCN, and mobile phase B of H₂O. LC-MS was performed to monitor and collect the pure product to obtain 18 mg of compound of formula (12) solid with a yield of 22%. LC-MS: (M+H)⁺1197.2.

### Example 2 Preparation of compound of formula (4)

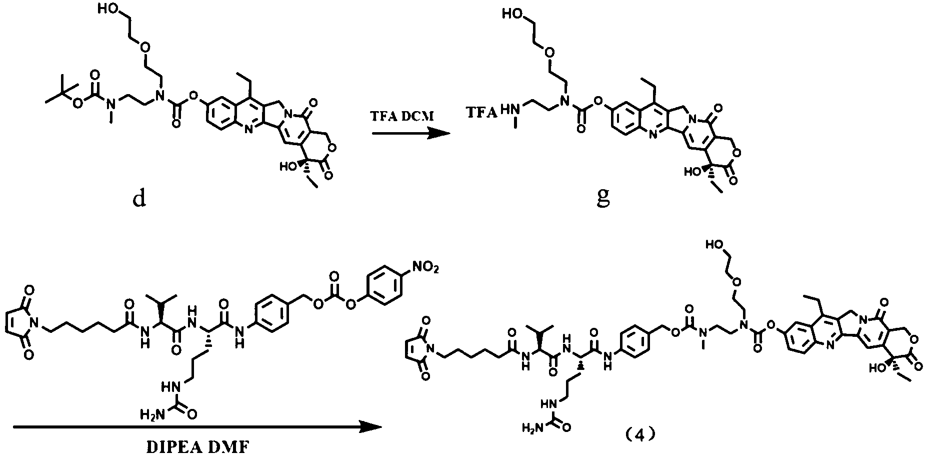

Compound g (10-(2-N-methylethyl ethylene glycol)-camptothecin trifluoroacetate) was prepared according to the synthetic route of Example 1: Compound d (73.0 mg) was dissolved in 3 mL of dichloromethane and 3 mL of trifluoroacetic acid, placed in an ice water bath, and stirred at constant temperature for 1 h. The reaction was detected to be completed by LC-MS. After the reaction was completed, the solvent was rotating-dried. The residue was added with 2 mL of dichloromethane, rotating-dried twice, and monitored by LC-MS to collect the product to obtain compound g (70 mg), which was directly used in the next reaction. LC-MS: (M+H)⁺ 580.6.

Compound of formula (4) (maleimidohexanoic acid-L-valyl-L-citrulline-p-aminobenzyl-(2-N-methylethyl ethylene glycol)-camptothecin) was prepared according to the synthetic route of Example 1: Mc-VC-PAB-PNP (maleimidohexanoic acid-L-valyl-L-citrulline-p-aminobenzyl-p-nitrophenyl carbonate) (58 mg) and compound g (42 mg) were dissolved in 2.5 mL of N,N-dimethylformamide, stirred in an ice water bath for 30 min, added dropwise with DIPEA (42 mg), and stirred in the ice water bath for 1 h. The reaction was detected to be uncompleted by LC-MS. The reaction system was added with triethylamine (10 mg) again and stirred at room temperature for 1 h. The reaction solution turned yellow, and the reaction was detected to be completed by LC-MS. After the reaction was completed, the solvent was rotating-dried at low temperature, and the residue was purified by preparative liquid chromatography using the preparative column of Sun Fire^{®} Prep C18 OBD TM 5µm, 19*250mm Column, mobile phase A of MeCN and 0.1% HCOOH, and mobile phase B of H₂O and 0.1% HCOOH. LC-MS was performed to monitor and collect the pure product to obtain 34 mg of compound of formula (4) solid with a yield of 40.5%. LC-MS: (M +H)⁺1179.2.

### Example 3 Preparation of compound of formula (1)

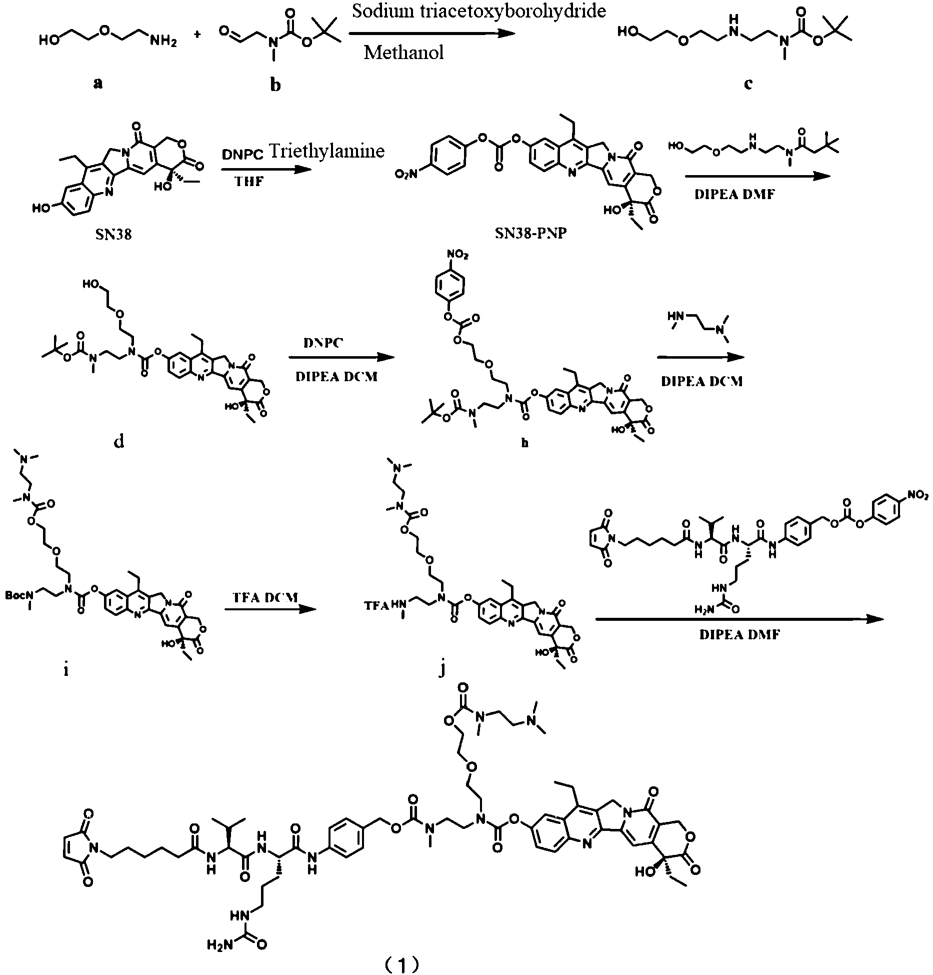

### 1) Synthesis of compound c (2-Boc-N-methylethyl ethylene glycol)

0.6 g of compound a (amino ethylene glycol) and 1.0 g of compound b (N-Boc-(methylamido)acetaldehyde) were dissolved in 20 mL of methanol, and stirred at room temperature for 6 h. The reaction system was placed in an ice water bath, added with sodium triacetoxyborohydride, stirred in the ice water bath for 1 h, then returned to room temperature and stirred overnight. The product was detected by LC-MS and showed no UV absorption. The solvent was rotating-dried, and the residue was extracted twice with ethyl acetate and dried over anhydrous Na₂SO₄. After pouring out the organic phase, the residue was rotating-dried, purified by column chromatography using dichloromethane: methanol of 20:1 as eluent, and rinsed to obtain 1.0 g of product with a yield of 66%. LC-MS: (M+H)⁺ 262.6.

### 2) Synthesis of SN38-PNP (10-p-nitrophenyl carbonate-camptothecin)

1.0 g of SN38 (camptothecin) and 1.6 g of DNPC (bis(p-nitrophenyl)carbonate) were dissolved in 100 mL of tetrahydrofuran, added with 2 mL of trimethylamine, and stirred at room temperature for 1.5 h. The reaction was detected to be completed by LC-MS. After the reaction was completed, the solvent was rotating-dried, the residue was triturated with ethyl acetate: n-hexane of 20 mL: 100 mL and filtered to obtain a solid. The solid was triturated with 20 mL of dichloromethane, filtered, and monitored by LC-MS to collect the product to obtain 700 mg of SN38-PNP solid with a yield of 49.3%. LC-MS: (M+H)⁺ 557.4.

### 3) Synthesis of compound d (Boc-N-methylethyl glycol-camptothecin)

500 mg of SN38-PNP was dissolved in 30 mL of N,N-dimethylformamide, added with 370 mg of compound c and 233 µL of N,N-diisopropylethylamine, and stirred at room temperature for 3 h. The reaction was detected to be completed by LC-MS. After the reaction was completed, the solvent was rotating-dried, and the residue was purified by column chromatography using dichloromethane: methanol of 20:1 as eluent, rinsed, and monitored by LC-MS to collect the product to obtain 250 mg of compound d solid with a yield of 45%. LC-MS: (M+H)⁺ 680.5.

### 4) Synthesis of compound h (Boc-N-methylethyldiethoxy p-nitro active ester-camptothecin)

220.0 mg of compound d was dissolved in 5 mL of dichloromethane, added with 150 mg of bis(p-nitrophenyl)carbonate and 86 mg of DIPEA, and stirred at constant temperature for 16 h. The reaction was detected to be completed by LC-MS. After the reaction was completed, the solvent was rotating-dried, and the residue was purified by column chromatography using dichloromethane: methanol of 50:1 as eluent. The product was collected to obtain 220 mg of compound h solid with a yield of 68.5%, which was then directly used in the next reaction. LC-MS: (M+H)⁺ 845.6.

### 5) Synthesis of compound i (Boc-N-methylethyldiethoxy-N,N,N-trimethylethylenediamine-camptothecin)

220 mg of compound h (Boc-N-methylethyldiethoxy p-nitroactive ester-camptothecin) and 50 mg of N,N,N-trimethylethylenediamine were dissolved in 5 mL of N,N-dimethylformamide, added dropwise with DIPEA (65 mg), and stirred in an ice water bath for 1 h. The reaction was detected to be completed by LC-MS. After the reaction was completed, the solvent was rotating-dried, and the residue was purified by column chromatography using dichloromethane: methanol of 20:1 as eluent. The product was collected to obtain 157 mg of compound i solid with a yield of 75%, which was then directly used in the next reaction. LC-MS: (M+H)⁺ 808.6.

### 6) Synthesis of compound j (N-methylethyldiethoxy-N,N,N-trimethylethylenediamine-camptothecin-trifluoroacetate)

157.0 mg of compound i (Boc-N-methylethyldiethoxy-N,N,N-trimethylethylenediamine -camptothecin) was dissolved in 3 mL of dichloromethane and 1.3 mL of trifluoroacetic acid, placed in an ice-water bath, and stirred at constant temperature for 1 h. The reaction was detected to be completed by LC-MS. After the reaction was completed, the solvent was rotating-dried. The residue was added with 2 mL of dichloromethane and then rotating-dried twice to obtain 120 mg of compound j, which was then directly used in the next reaction. LC-MS: (M+H)⁺ 708.3.

### 7) Synthesis of compound of formula (1) (maleimidohexanoic acid-L-valyl-L-citrulline-p-aminobenzyl-(N-methylethyldiethoxy-N,N,N-trimethylethylenediamine)-camptothecin)

52 mg of Mc-VC-PAB-PNP (maleimidohexanoic acid-L-valyl-L-citrulline-p-aminobenzyl-p-nitrophenyl carbonate) and 35 mg of compound j were dissolved in 2.5 mL of N,N-dimethylformamide, stirred in an ice water bath for 30 min, added dropwise with 129 mg of DIPEA, and stirred in the ice water bath for 0.5 h. The reaction was detected to be uncompleted by LC-MS. The reaction system was then added with 129 mg of DIPEA and stirred at room temperature for 0.5 h. The reaction solution turned yellow, and the reaction was detected to be completed by LC-MS. After the reaction was completed, the solvent was rotating-dried at low temperature, and the residue was purified by preparative liquid chromatography using the preparative column of Sun Fire^{®} Prep C18 OBD TM 5µm, 19*250mm Column mobile phase A of MeCN and 0.1% HCOOH, and mobile phase B of H₂O and 0.1% HCOOH. LC-MS was performed to monitor and collect the pure product to obtain 20 mg of compound of formula (1) solid with a yield of 20.5%. LC-MS: (M+H)⁺ 1307.6.

### Example 4 Preparation of compound of formula (9)

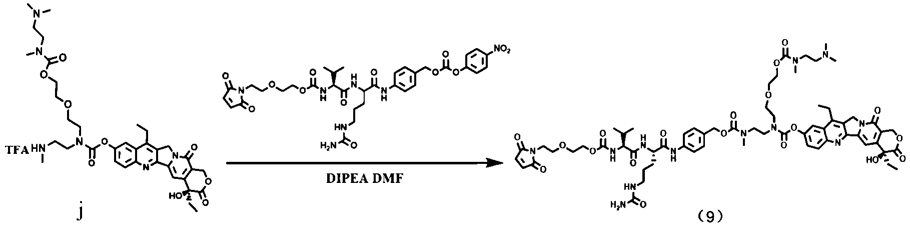

Compound of formula (9) (maleimidediethoxy-L-valyl-L-citrulline-p-aminobenzyl-(N-methylethyldiethoxy-N,N,N-trimethylethylenediamine)-camptothecin) was prepared according to the synthetic route of Example 3: MP2-VC-PAB-PNP (maleimidediethoxy-L-valyl-L-citrulline-p-aminobenzyl-p-nitrophenyl carbonate) (52 mg) and compound j (34 mg) were dissolved in 2.5 mL of N,N-dimethylformamide, stirred for 30 min in an ice water bath, added dropwise with DIPEA (36 mg), and stirred in the ice water bath for 0.5 h. The reaction was detected to be uncompleted by LC-MS. The reaction system was then added with DIPEA (8 mg) and stirred at room temperature for 1.5 h. The reaction solution turned yellow, and the reaction was detected to be completed by LC-MS. After the reaction was completed, the solvent was rotating-dried at low temperature, and the residue was purified by preparative liquid chromatography using the preparative column of Sun Fire^{®} Prep C18 OBD TM 5µm, 19*250mm Column, mobile phase A of MeCN and 0.1% HCOOH, and mobile phase B of H₂O and 0.1% HCOOH. LC-MS was performed to monitor and collect the pure product to obtain 23 mg of compound of formula (9) solid with a yield of 35.2%. LC-MS: (M+H)⁺ 1325.4.

### Example 5 Inhibitory effect of ADC-1 on NCI-N87 cells

Compound of formula (1) and CL2A-SN38 intermediate were subjected to sulfhydryl coupling with Heceptin by conventional methods in the art, respectively (for example, see Example 12 in the Chinese Patent Application Publication No. CN102448494A), to obtain ADC-1 and HER2-CL2A-SN38 (ADC-17) antibody-drug conjugates with an average DAR value of 8. NCI-N87 cells were digested by trypsin, then adjusted to a cell density of 50,000 cells/mL, added to a cell culture plate at 100 µL/well, and cultured in a 37°C, 5% CO₂ incubator for 14-20 h. The sample and control groups (see Table 1 for the composition of the test samples) were gradiently diluted with basal medium, respectively, transferred to the cell culture plate inoculated with cells at 100 µL/well, and cultured in a 37°C, 5% CO₂ incubator for 70-74 h. CCK-8 was diluted 10 times with culture medium. The old medium in the 96-well plate was discarded, and 100 µL of diluted CCK-8 solution was added to each well. Color development was performed under 5% CO₂ for 2-4 h. After centrifugation to remove air bubbles, the plates were detected by a microplate reader at a detection wavelength of 450 nm/655 nm for reading. The results are shown in Table 2.

**Table 1 Composition of test samples**

| Number | Test sample | Average DAR |
|---|---|---|
| Sample 1 | ADC-1 | 8 |
| Control group 1 | ADC-17 | 8 |
| Control group 2 | HER2 antibody | / |

**Table 2 Inhibitory effect on NCI-N87 cells**

| Number | Test sample | IC50 (ng/ml) |
|---|---|---|
| Sample 1 | ADC-1 | 251 |
| Control group 1 | ADC-17 | 210 |
| Control group 2 | HER2 antibody | NA |

The experimental results show that the ADC-1 group and the ADC-17 group had comparable inhibitory effect on NCI-N87 cells.

### Example 6 Experimental research on stability

1) Compound of formula (1) (2.1 mg) and CL2A-SN38 (2.1 mg) were precisely weighed, and then dissolved in 700 µL of DMSO, respectively.
2) PB (0.2 M) buffers at pH of 6, 7 and 8 were prepared, respectively.
3) 50 µL of DMSO solution of compound of formula (1) was added in a centrifuge tube, and added with 950 µL of PB (0.2 M) buffer at pH of 6, where the compound had a concentration of 60 µg/mL. The system was placed in a constant temperature system of 37°C. 50 µL of DMSO solution of CL2A-SN38 was added in a centrifuge tube, and added with 950 µL of PB (0.2 M) buffer at pH of 6, where the compound had a concentration of 60 µg/mL. The system was placed in a constant temperature system of 37°C;
4) 40 µL of sample was respectively taken at 0 h, 2 h, 4 h, 6 h, 24 h, and 48 h, added with 120 µL of acetonitrile, and centrifuged. The supernatant was taken for quantitative determination by LC-MS.
5) Results processing.

The concentration at T=0 h in LC-MS was used as the reference, and the data were calculated by T/T₀*100%. The results are shown in Table 3 and Table 4, respectively.

**Table 3 Compound of formula (1)**

| Time | pH=6 | pH=7 | pH=8 |
|---|---|---|---|
| 0 | 1 | 1 | 1 |
| 2 | 0.988606 | 0.878866 | 0.56405 |
| 4 | 0.97612 | 0.801849 | 0.284131 |
| 6 | 0.92084 | 0.821934 | 0.123407 |
| 24 | 0.86821 | 0.256709 | 0.009607 |
| 48 | 0.78862 | 0.122173 | 0.012562 |

At pH of 6, the compound of formula (1) decomposed by 21.2% after 48 h;
At pH of 7, the compound of formula (1) decomposed by 87.8% after 48 h;
At pH of 8, the compound of formula (1) decomposed by 98.7% after 48 h.

**Table 4 CL2A-SN38**

| Time | pH=6 | pH=7 | pH=8 |
|---|---|---|---|
| 0 | 1 | 1 | 1 |
| 2 | 0.747874 | 0.703812 | 0.628692 |
| 4 | 0.647065 | 0.585106 | 0.434695 |
| 6 | 0.412955 | 0.571543 | 0.38435 |
| 24 | 0.035931 | 0.022606 | 0.025316 |
| 48 | 0.020243 | 0.01906 | 0.019275 |

At pH of 6, CL2A-SN38 decomposed by 98.0% after 48 h;
At pH of 7, CL2A-SN38 decomposed by 98.1% after 48 h;
At pH of 8, CL2A-SN38 decomposed by 98.1% after 48 h.

From the results in Table 3 and Table 4, it can be seen:
1) Under weak acidic conditions, the compound of formula (1) was more stable than CL2A-SN38.
2) Under neutral conditions, the compound of formula (1) decomposed by 18% at 6 h and 75% at 24 h, and CL2A-SN38 decomposed by 43% at 6 h and 98% at 24 h.
3) Under alkaline conditions, the compound of formula (1) decomposed by 97% at 24 h, and CL2A-SN38 decomposed by 98% at 24 h.

Therefore, compared with CL2A-SN38, the compound of formula (1) prepared in the present disclosure had higher stability under weak acidic or neutral conditions *in vivo,* which is expected to significantly improve the safety of ADC drugs.

### Example 7 Assessment of inhibitory effect of anti-Her2-drug conjugates on the development of in situ breast cancer

### 1. Experimental methods

BT474 cell line was thawed and passaged for 1-2 times. After the cells grew in a stable state, they were cultured on expansion. A suspension of tumor cells was prepared. Nude mice were inoculated with 0.2*10⁷ BT474 cells in mammary fat pads, and a total of 60 female BALB/c nude mice were inoculated. When the average tumor volume reached about 70 mm³, 42 mice were selected and randomly divided into 6 groups according to the tumor volume: negative control group as control group 1 (normal saline group), control group 2 (ADC-17), experimental group 1 (ADC-4), experimental group 2 (ADC-12), experimental group 3 (ADC-1), and experimental group 4 (ADC-9), 7 mice in each group. After the model was successfully established, according to the above grouping, control group 1 was intravenously given normal saline, and the remaining groups were intravenously given drugs at doses of 5 mg/kg for control group 2, 5 mg/kg for experimental group 1, 5 mg/kg for experimental group 2, 5 mg/kg for experimental group 3, and 5 mg/kg for experimental group 4, once a week for 21 weeks. The mice were weighed and the tumor volumes were measured 3 times a week, and the mice were weighed and the tumor volumes were measured on day 21.

### 2. Experimental results and analysis

### 1) Changes in body weight of mice:

**Table 5 Body weight of mice**

| Group | | Number | | Weight (g) | |
|---|---|---|---|---|---|
| | | D1 | D21 | D1 | D21 |
| Control group 1 | Normal saline | 7 | 7 | 18.40±1.41 | 19.94±1.22 |
| Control group 2 | ADC-17 | 7 | 7 | 18.73±0.78 | 20.36±0.90 |
| Experimental group 1 | ADC-4 | 7 | 7 | 18.43±0.95 | 20.42±0.86 |
| Experimental group 2 | ADC-12 | 7 | 7 | 18.43±0.95 | 19.94±0.72 |
| Experimental group 3 | ADC-1 | 7 | 7 | 17.88±1.01 | 18.64±1.31 |
| Experimental group 4 | ADC-9 | 7 | 7 | 18.13±1.14 | 18.61±1.04 |

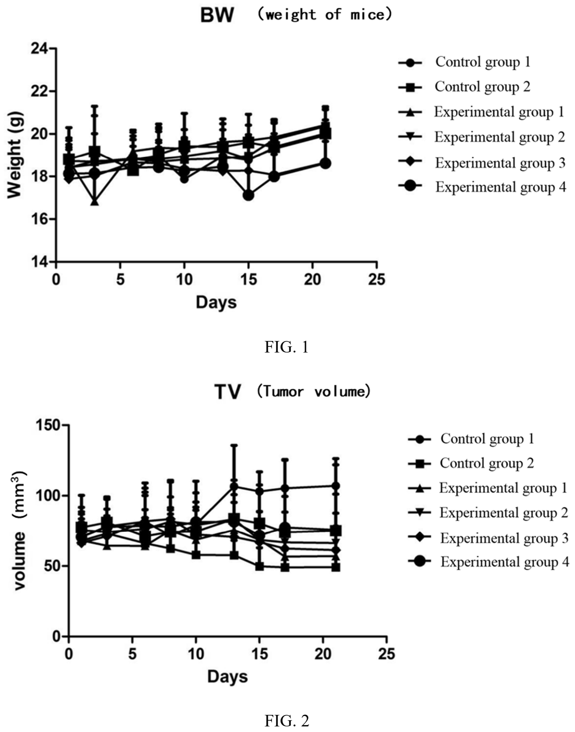

No mice died during the experiment. At the end of the experiment, each group of control group 2 (ADC-17 group), experimental group 1 (ADC-4), experimental group 2 (ADC-12), experimental group 3 (ADC-1), and experimental group 4 (ADC-9) showed no significant difference in body weight compared with control group 1. See Table 5 and FIG. 1 for details.

### 2) Tumor volume of mice:

**Table 6 Tumor volume of mice**

| Group | | Tumor volume (mm³) | | RTV | T/C (%) |
|---|---|---|---|---|---|
| | | D1 | D21 | | |
| Control group 1 | Normal saline | 75.60±24.66 | 107.07±19.30^{##} | 1.52±0.39^{##} | - |
| Control group 2 | ADC-17 | 67.95±15.73 | 49.17±13.29^{**} | 0.75±0.19** | 49.30 |
| Experimental group 1 | ADC-4 | 68.24±13.13 | 57.12±20.28^{**} | 0.83±0.27** | 55.00 |
| Experimental group 2 | ADC-12 | 70.73±16.36 | 66.26±21.37^{**} | 0.95±0.26* | 62.58 |
| Experimental group 3 | ADC-1 | 66.47±14.09 | 61.32±12.82^{**} | 0.95±0.23** | 62.48 |
| Experimental group 4 | ADC-9 | 70.73±17.99 | 75.76±46.18^{*} | 1.02±0.35* | 67.19 |

| | | | | | |
|---|---|---|---|---|---|
| Note: Each group was compared with control group 1 of normal saline group, where "*" represents P <0.05 and "^{**}" represents P<0.01; and each group was compared with control group 2, where "^{#}" repr esents P<0.05 and "^{##}" represents P<0.01. | | | | | |

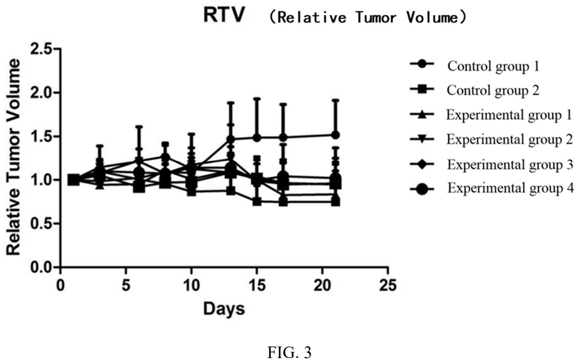

The tumor volume of control group 1 reached 107±19.30 mm³ at Day 21 after administration, and the tumor volumes of control group 2, experimental group 1, experimental group 2, experimental group 3, and experimental group 4 were 49.17±13.29, 57.12±20.28, 66.26±21.37, 61.32±12.82, 75.76±46.18, and 75.19±25.99 mm³ respectively (see Table 6). Among them, control group 2, experimental group 1, experimental group 2 and experimental group 3 were statistically very significantly different (P<0.01) from control group 1 (normal saline group), and experimental group 4 was statistically significantly different (P<0.05) from control group 1. The tumor volumes of experimental group 1, experimental group 2, and experimental group 3 were not statistically significantly different (P>0.05) from that of control group 2, and their relative tumor volumes were also not significantly different (P > 0.05), *i.e.,* the effects were comparable. Among them, the relative tumor proliferation rates (T/C) of control group 2, experimental group 1, experimental group 2, experimental group 3, and experimental group 4 were 49.30%, 55.00%, 62.58%, 62.48%, 67.19%, and 63.20%, respectively. From the above data, it can be known that all of the four antibody-drug conjugates corresponding to control group 2, experimental group 1, experimental group 2 and experimental group 3 tested in this experiment significantly inhibited the BT474 tumor growth with comparable effects, and experimental group 4 also inhibited the BT474 tumor growth. The changes in tumor volume and relative tumor volume of each group during the experiment are shown in FIG. 2 and FIG. 3, respectively.

In summary, the ADCs constructed with the drug conjugate intermediates of the present disclosure showed significant antitumor activity against the breast cancer BT474 cell model. The preferable ADC-1, ADC-4, and ADC-12 had significant advantages and were expected to significantly improve safety under *in vivo* conditions while providing tumor inhibitory effects comparable to that of the ADC-17 group.

The present disclosure has been exemplified by various specific examples. However, a person of ordinary skill in the art can understand that the present disclosure is not limited to each specific embodiments, and a person of ordinary skill can make various changes or modifications within the scope of the present disclosure, and each technical feature mentioned in various places in this specification can be combined with each other without departing from the spirit and scope of the present disclosure. Such changes and modifications are within the scope of the present disclosure.

## Claims

1. An antibody drug conjugate selected from the following structures: 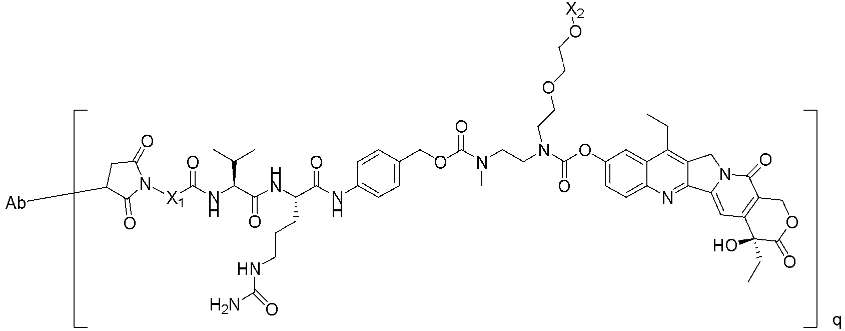 wherein:
X₁ is an alkane chain or a PEG chain,
X₂ is H or -C(O)NR¹R², and
R¹ is selected from the group consisting of hydrogen, halogen, hydroxyl, substituted or unsubstituted C₁₋₆ alkyl, substituted or unsubstituted C₁₋₆ hydroxyalkyl, substituted or unsubstituted C₁₋₆ aminoalkyl, substituted or unsubstituted C₁₋₆ alkoxyl, substituted or unsubstituted C₁₋₆ alkylacyl, and substituted or unsubstituted C₁₋₆ alkylaldehyde;
R² is selected from the group consisting of hydrogen, 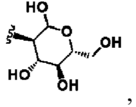 and substituted or unsubstituted C₁₋₆ alkyl; wherein each substituted C₁₋₆ alkyl is optionally substituted with 1-5 substituents independently selected from the group consisting of halogen, hydroxyl, amino, carbonyl, carboxyl, 5-10-membered heterocyclyl and C₁₋₆ haloalkyl, wherein the 5-10-membered heterocyclyl has 1-3 heteroatoms selected from the group consisting of oxygen, nitrogen and sulfur;
Ab is antibody;
q is selected from 1, 2, 3, 4, 5, 6, 7, and 8.

2. The antibody-drug conjugate according to claim 1, wherein X₁ is selected from the group consisting of
**-(CH₂)**ₘ- and -(**CH₂CH₂**O)ₚ-,
wherein:
m is selected from 1, 2, 3, 4, 5, and 6;
p is selected from 1, 2, 3, 4, 5, and 6.

3. The antibody-drug conjugate according to claim 2, wherein m is 5, and p is 2.

4. The antibody-drug conjugate according to claim 2, wherein X₁ is selected from the group consisting of:

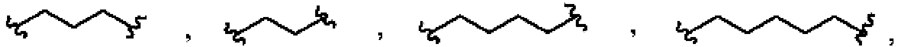

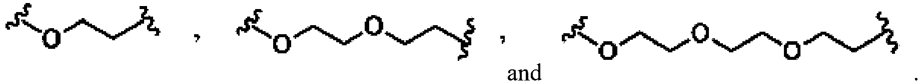

5. The antibody-drug conjugate according to claim 1, wherein R¹ or R² is independently selected from the group consisting of: H, methyl, ethyl, propyl, butyl, pentyl, heptyl, methoxyl, ethoxyl, Cl, Br,

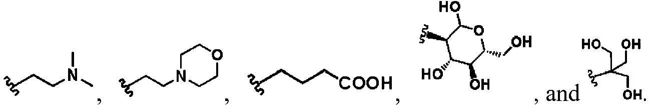

6. The antibody-drug conjugate according to claim 1, wherein X₂ is selected from the group consisting of:

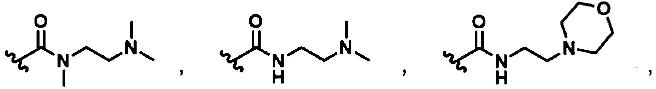

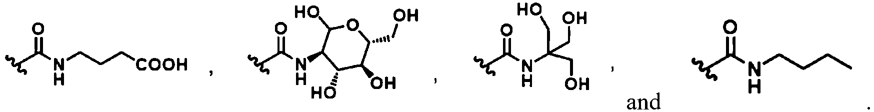

7. The antibody-drug conjugate according to claim 1, which is one selected from the group consisting of formula (ADC-1) to ( ADC-16):
| | |
|---|---|
| 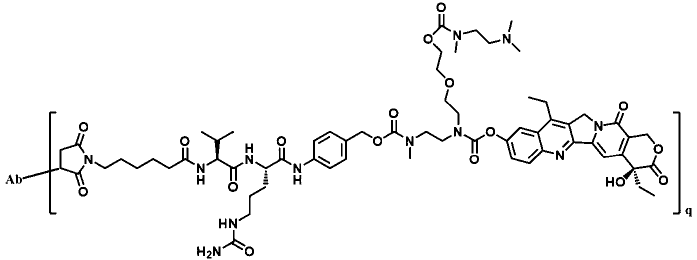 | ADC-1 |
| 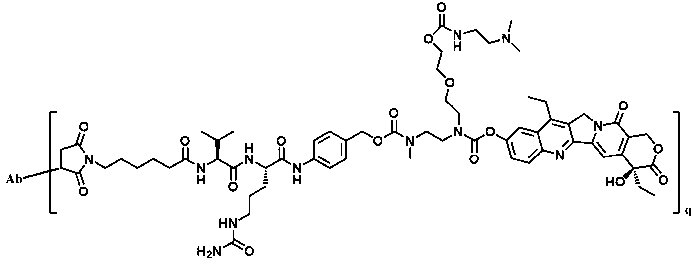 | ADC-2 |
| 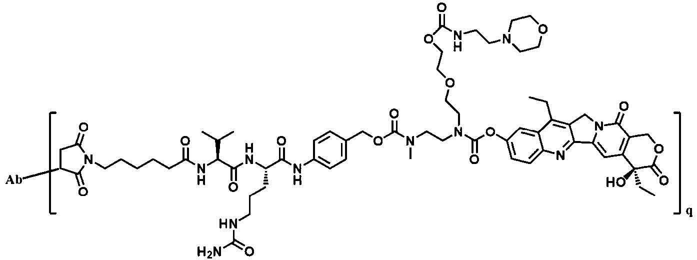 | ADC-3 |
| 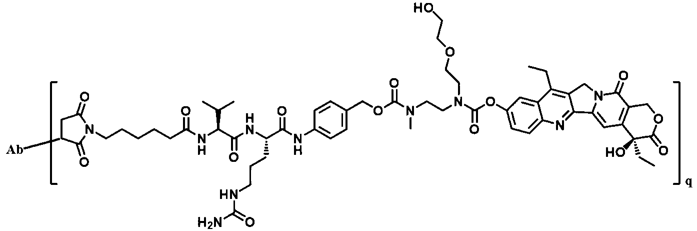 | ADC-4 |
| 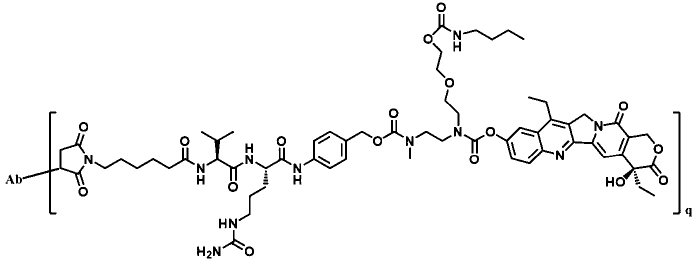 | ADC-5 |
| 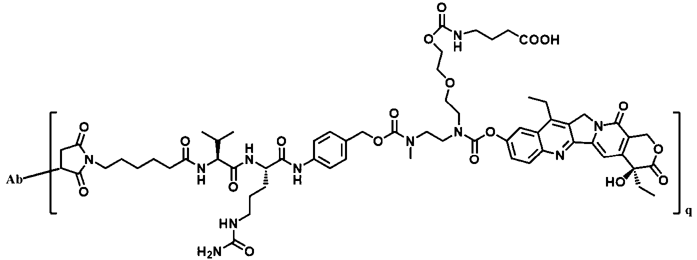 | ADC-6 |
| 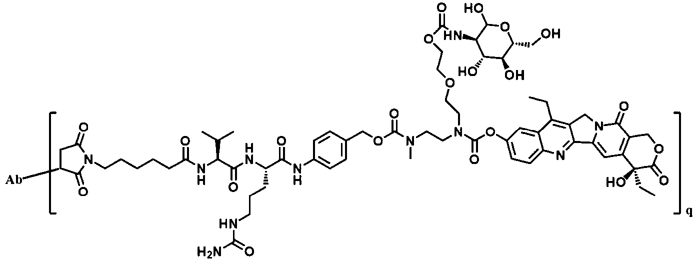 | ADC-7 |
| 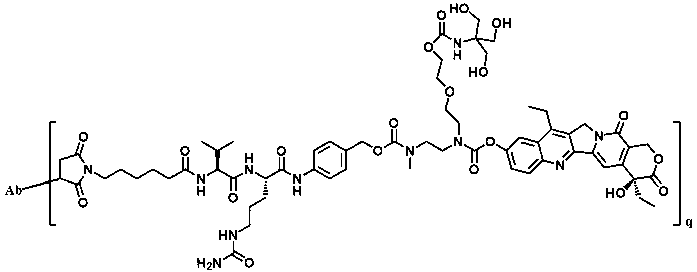 | ADC-8 |
| 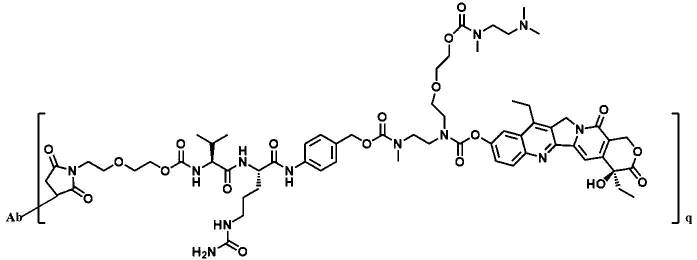 | ADC-9 |
| 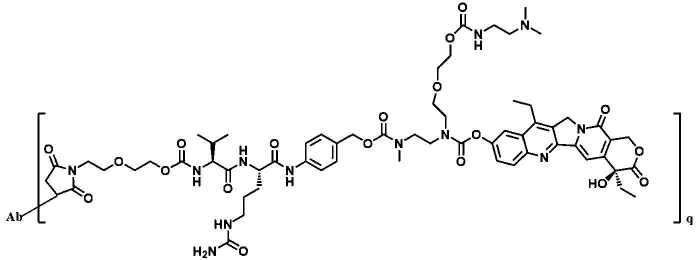 | ADC-10 |
| 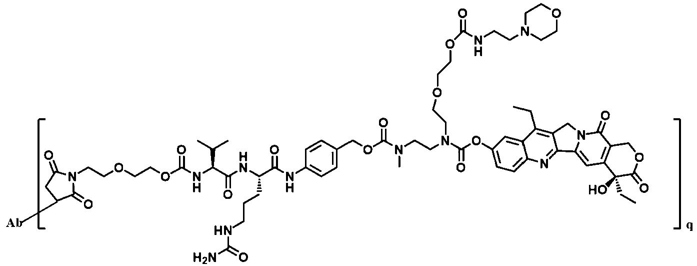 | ADC-11 |
| 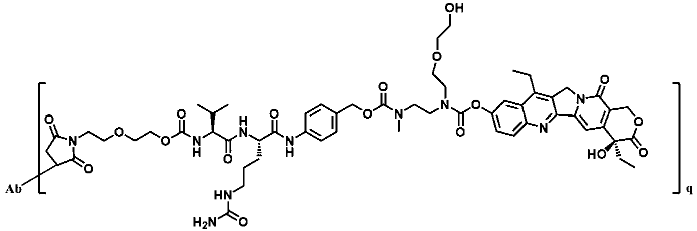 | ADC-12 |
| 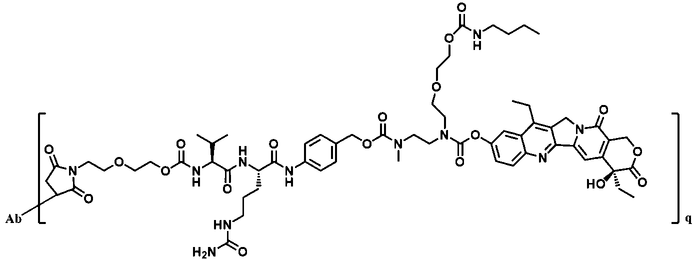 | ADC-13 |
| 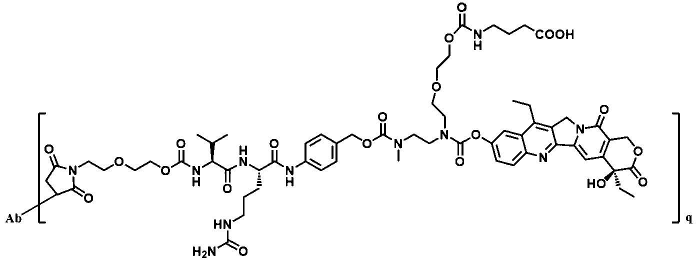 | ADC-14 |
| 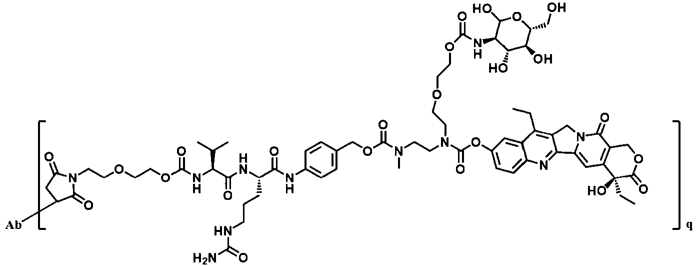 | ADC-15 |
| 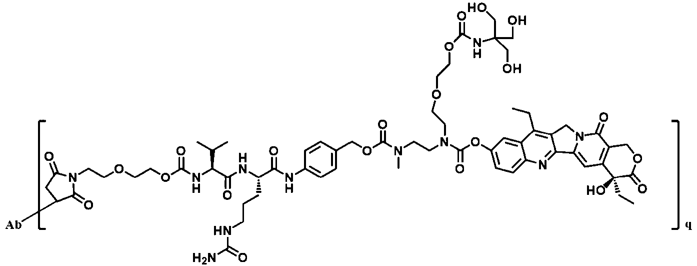 | ADC-16 |

8. A pharmaceutical composition comprising the antibody drug conjugate of any one of claims 1-7 for use in treating a tumor.
